# EUROPEAN PATENT APPLICATION

(11) **EP 4 588 921 A1**
(43) Date of publication of application: **23.07.2025**
(21) Application number: 23864748.1
(22) Date of filing: 14.09.2023
(51) Int. Cl.: C07D 487/04, A61K 31/407, A61P 25/00

(54) **FREE BASE CRYSTAL FORM OF POLYCYCLIC COMPOUND OF NITROGEN-CONTAINING HETEROCYCLE, AND PREPARATION METHOD THEREFOR**

(30) Priority: 14.09.2022 CN 202211116574
(71) Applicant: Jiangsu Hansoh Pharmaceutical Group Co., Ltd., Lianyungang, Jiangsu 222047 (CN); Shanghai Hansoh Biomedical Co., Ltd., Shanghai 201203 (CN)
(72) Inventor: YANG, Tong, Shanghai 201203 (CN); CHEN, Jinyao, Shanghai 201203 (CN)
(74) Representative: Cabinet Beau de Loménie
(86) International application number: PCT/CN2023/118743
(87) International publication number: WO 2024/056016

(57) **Abstract**

The present invention relates to a free base crystal form of a polycyclic compound of a nitrogen-containing heterocycle, and a preparation method therefor. Specifically, the present invention provides a free base crystal form of a compound having general formula (I), a preparation method therefor, a pharmaceutical composition containing a therapeutically-effective amount of the crystal form, and a use thereof as an orexin receptor antagonist in the preparation of drugs for treating nervous system diseases.

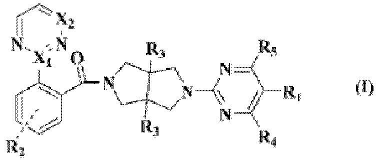

## Description

### Technical Field

The present invention belongs to the field of drug synthesis, and specifically relates to a crystal form of a polycyclic compound of a nitrogen-containing heterocycle, and a preparation method therefor and the use thereof.

### Background Art

The hypothalamus is the centre for the regulation of diet and energy balance. Orexin is a neuropeptide synthesized and secreted by orexin neurons in the lateral hypothalamus (LH) and is named for its strong appetite-promoting effect. Orexin is divided into orexin A and orexin B. Both orexin A and orexin B act on the G protein-coupled receptors, orexin receptor OX1R and orexin receptor OX2R. OX1R and OX2R are widely expressed throughout the central nervous system. In addition, OX1R has a stronger binding ability with orexin A than orexin B, whereas OX2R has a comparable binding ability with orexin A and orexin B. Orexin has a complex relationship with other neuropeptides that affect eating, and has a wide range of effects on increasing food intake, drinking water, regulating sleep-wake cycles, reproduction, body temperature, blood pressure, sensation, etc. For example, orexin regulates wakefulness and wakefulness by regulating two different G protein-coupled receptors OX1R and OX2R. Orexin receptor antagonist has a potential therapeutic advantage in the treatment of nervous system diseases, including insomnia, depression, anxiety, drug addiction, etc.

OX1R and OX2R activate intracellular Ca²⁺ by phospholipase C. OX2R can also couple Gi/Go and inhibit the production of cAMP by inhibiting adenylate cyclase. Studies have found that among OX1R and OX2R, OX2R is preferentially expressed in the paraventricular nucleus of the hypothalamus and participates in the regulation of the hypothalamic-pituitary-adrenal (HPA) axis. The nocturnal HPA overactivity is the biggest difference between depression patients and normal people. Downregulation of the HPA system overactivity may help improve depressive symptoms.

Currently, there are multiple drugs targeting OX1/2R that are in the clinical stages or have been on the market, such as Suvoraxant, Merck and Lemborexant, Eisai. However, drugs as orexin 1/2 antagonists have antagonistic effects on both OX1R and OX2R receptors. The antagonistic effect on OX1R may change the normal physiological structure of rapid eye movement sleep (NEM, the brain activity is the same as when awake) and non-rapid eye movement sleep (NEREM, i.e., deep sleep), that is, NREM time is sacrificed and REM time is prolonged, so that, the risk of sleepiness is increased and OX1R antagonists cannot have an antidepressant effect.

OX2R antagonists can have an antidepressant effect and the receptor antagonist targeting only OX2R can also have sufficient efficacy for insomnia, so that the selective OX2R antagonist can avoid multiple side effects caused by the effect on OX1R, such as sleepiness. Currently, among OX2R antagonists, only Seltorexant, developed by Janssen is in the clinical stage, and the main indications thereof are major depressive disorder (MDD), primary and secondary insomnia, etc.

Selective OX2R antagonists have the potential to treat nervous system diseases, such as insomnia, depression and anxiety, and there is a huge clinical need for selective OX2R antagonists. Selective OX2R antagonists as drugs have a good application prospect in the pharmaceutical industry.

The PCT patent (application number: PCT/CN2022/080829) discloses the structures of a series of polycyclic compounds of nitrogen-containing heterocycle. In subsequent research and development, in order to make the product easy to handle, filter and dry, and to find a suitable crystal that is easy to store and has long-term stability, the present invention conducts a comprehensive study on the free base crystal form of the above-mentioned compounds.

### Summary of the Invention

All contents involved in patent PCT/CN2022/080829 are added to the present invention by reference.

An objective of the present invention is to provide a crystal form of a compound represented by general formula (I), wherein the crystal form has a structure as represented by formula (I): 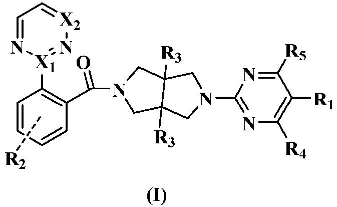 wherein
X₁ is CR₆ or N;
X₂ is CR₆ or absent;
R₁ is halogen;
R₂, R₃, R₄ or R₆ is independently selected from hydrogen, deuterium, halogen, amino, nitro, hydroxyl, mercapto, cyano, carboxyl, sulfo, oxo, thio, alkyl, deuteroalkyl, haloalkyl, hydroxyalkyl, alkoxy, haloalkoxy, hydroxyalkoxy, alkenyl, alkynyl, cycloalkyl, heterocyclyl, aryl or heteroaryl, respectively, wherein the amino, alkyl, deuteroalkyl, haloalkyl, hydroxyalkyl, alkoxy, haloalkoxy, hydroxyalkoxy, alkenyl, alkynyl, cycloalkyl, heterocyclyl, aryl and heteroaryl can be optionally further substituted;
R₂, R₃, R₄ or R₆ is each independently preferably hydrogen, deuterium, halogen, amino, nitro, hydroxyl, mercapto, cyano, carboxyl, sulfo, oxo, thio, C₁₋₈ alkyl, C₁₋₈ deuteroalkyl, C₁₋₈ haloalkyl, C₁₋₈ hydroxyalkyl, C₁₋₈ alkoxy, C₁₋₈ haloalkoxy, C₁₋₈ hydroxyalkoxy, C₂₋₈ alkenyl, C₂₋₈ alkynyl, C₃₋₁₂ cycloalkyl, 3- to 12-membered heterocyclyl, C₆₋₁₄ aryl or 5- to 14-membered heteroaryl, wherein the amino, C₁₋₈ alkyl, C₁₋₈ deuteroalkyl, C₁₋₈ haloalkyl, C₁₋₈ hydroxyalkyl, C₁₋₈ alkoxy, C₁₋₈ haloalkoxy, C₁₋₈ hydroxyalkoxy, C₂₋₈ alkenyl, C₂₋₈ alkynyl, C₃₋₁₂ cycloalkyl, 3- to 12-membered heterocyclyl, C₆₋₁₄ aryl and 5- to 14-membered heteroaryl are optionally further substituted with one or more substituents of deuterium, halogen, amino, nitro, hydroxyl, mercapto, cyano, carboxyl, sulfo, oxo, thio, C₁₋₈ alkyl, C₁₋₈ deuteroalkyl, C₁₋₈ haloalkyl, C₁₋₈ hydroxyalkyl, C₁₋₈ alkoxy, C₁₋₈ haloalkoxy, C₂₋₈ alkenyl, C₂₋₈ alkynyl, C₃₋₁₂ cycloalkyl, 3- to 12-membered heterocyclyl, C₆₋₁₄ aryl and 5- to 14-membered heteroaryl,
more preferably hydrogen, deuterium, halogen, amino, nitro, hydroxyl, mercapto, cyano, carboxyl, sulfo, oxo, thio, C₁₋₆ alkyl, C₁₋₆ deuteroalkyl, C₁₋₆ haloalkyl, C₁₋₆ hydroxyalkyl, C₁₋₆ alkoxy, C₁₋₆ haloalkoxy, C₁₋₆ hydroxyalkoxy, C₂₋₆ alkenyl, C₂₋₆ alkynyl, C₃₋₈ cycloalkyl, 3- to 8-membered heterocyclyl, C₆₋₁₀ aryl or 5- to 10-membered heteroaryl, wherein the amino, C₁₋₆ alkyl, C₁₋₆ deuteroalkyl, C₁₋₆ haloalkyl, C₁₋₆ hydroxyalkyl, C₁₋₆ alkoxy, C₁₋₆ haloalkoxy, C₁₋₆ hydroxyalkoxy, C₂₋₆ alkenyl, C₂₋₆ alkynyl, C₃₋₈ cycloalkyl, 3- to 8-membered heterocyclyl, C₆₋₁₀ aryl and 5- to 10-membered heteroaryl are optionally further substituted with one or more substituents of deuterium, halogen, amino, nitro, hydroxyl, mercapto, cyano, carboxyl, sulfo, oxo, thio, C₁₋₆ alkyl, C₁₋₆ deuteroalkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₈ cycloalkyl, 3- to 8-membered heterocyclyl, C₆₋₁₀ aryl and 5- to 10-membered heteroaryl;
alternatively, any two or more of R₂, R₃ and R₄ and the atom to which they are attached are connected to form cycloalkyl, heterocyclyl, aryl or heteroaryl, wherein the cycloalkyl, heterocyclyl, aryl and heteroaryl can be optionally further substituted;
R₅ is selected from hydroxyalkyl or haloalkyl, which can be optionally further substituted, wherein the hydroxyalkyl is preferably 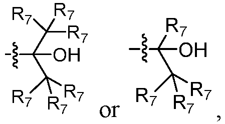 and the haloalkyl is preferably 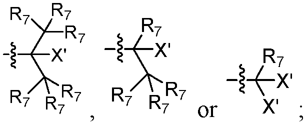
X' is halogen;
R₇ is independently selected from hydrogen, deuterium, halogen, amino, nitro, hydroxyl, mercapto, cyano, carboxyl, sulfo, oxo, thio, alkyl, deuteroalkyl, haloalkyl, hydroxyalkyl, alkoxy, haloalkoxy, hydroxyalkoxy, alkenyl, alkynyl, cycloalkyl, heterocyclyl, aryl or heteroaryl, respectively, wherein the amino, alkyl, deuteroalkyl, haloalkyl, hydroxyalkyl, alkoxy, haloalkoxy, hydroxyalkoxy, alkenyl, alkynyl, cycloalkyl, heterocyclyl, aryl and heteroaryl can be optionally further substituted, preferably, hydrogen, deuterium, alkyl, deuteroalkyl, haloalkyl, hydroxyalkyl, alkoxy, haloalkoxy or hydroxyalkoxy, which can be optionally further substituted with one or more substituents of deuterium, halogen, C₁₋₆ alkyl, C₁₋₆ deuteroalkyl, C₁₋₆ haloalkyl, C₁₋₆ hydroxyalkyl, C₁₋₆ alkoxy and C₁₋₆ haloalkoxy, further preferably, hydrogen, deuterium, C₁₋₆ alkyl, C₁₋₆ deuteroalkyl, C₁₋₆ haloalkyl, C₁₋₆ hydroxyalkyl, C₁₋₆ alkoxy and C₁₋₆ haloalkoxy, more further preferably, hydrogen, deuterium, C₁₋₃ alkyl, C₁₋₃ deuteroalkyl, C₁₋₃ haloalkyl, C₁₋₃ hydroxyalkyl, C₁₋₃ alkoxy and C₁₋₃ haloalkoxy, more preferably, hydrogen, deuterium, and C₁₋₃ alkyl.

In a preferred embodiment of the present invention, provided is a crystal form of a compound as represented by general formula (II), 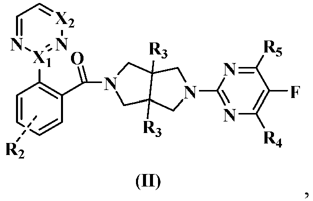 wherein
X₁ is CH or N;
X₂ is CH or absent;
R₂, R₃ or R₄ is independently selected from hydrogen, deuterium, halogen, amino, hydroxyl, C₁₋₃ alkyl, C₁₋₃ deuteroalkyl, C₁₋₃ haloalkyl, C₁₋₃ hydroxyalkyl, C₁₋₃ alkoxy, C₁₋₃ haloalkoxy or C₁₋₃ hydroxyalkoxy, respectively;
R₅ is selected from 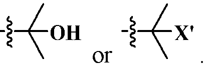
when X₂ is absent, substituent R₂ is preferably halogen, wherein the halogen is preferably located at an ortho or meta position of triazole, more preferably at an ortho position of triazole, and
the halogen is preferably fluorine;
when X₂ is CR₆, substituent R₂ is preferably halogen, wherein the halogen is preferably located at an ortho or meta position of pyrimidine, more preferably at a meta position of pyrimidine, and/or at an ortho position of carbonyl, and
the halogen is preferably fluorine.

In a preferred embodiment of the present invention, the general formula (I) is selected from the following compounds: 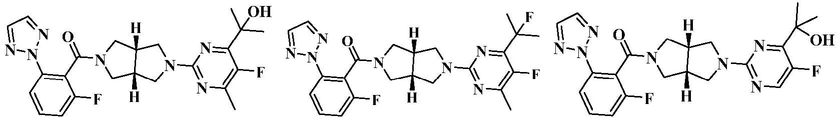 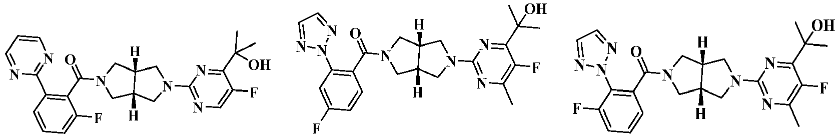 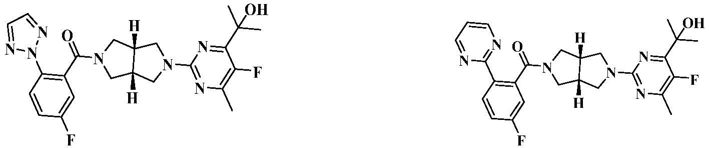 and

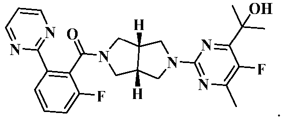

In a preferred embodiment of the present invention, provided is a crystal form of ((3aR,6aS)-5-(5-fluoro-4-(2-hydroxyprop-2-yl)pyrimidin-2-yl)hexahydropyrrolo [3,4-c]pyrrol-2(1H)-yl)(2-fluoro-6-(pyrimidin-2-yl)phenyl)methanone (compound 9), wherein the compound has the following structure:

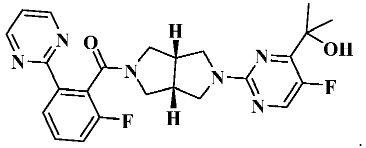

The X-ray powder diffraction pattern of the crystal form I has a diffraction peak at 2θ of 8.6±0.2°, or has a diffraction peak at 2θ of 9.2±0.2°, or has a diffraction peak at 2θ of 10.2±0.2°, or has a diffraction peak at 2θ of 10.8±0.2°, or has a diffraction peak at 2θ of 12.0±0.2°, or has a diffraction peak at 2θ of 12.8±0.2°, or has a diffraction peak at 2θ of 13.7±0.2°, or has a diffraction peak at 2θ of 14.2±0.2°, or has a diffraction peak at 2θ of 15.5±0.2°, or has a diffraction peak at 2θ of 16.6±0.2°, or has a diffraction peak at 2θ of 17.3±0.2°, or has a diffraction peak at 2θ of 18.4±0.2°, or has a diffraction peak at 2θ of 19.0±0.2°, or has a diffraction peak at 2θ of 19.5±0.2°, or has a diffraction peak at 2θ of 20.5±0.2°, or has a diffraction peak at 2θ of 21.3±0.2°, or has a diffraction peak at 2θ of 26.5±0.2°; or has a diffraction peak at 2θ of 28.6±0.2°; preferably, the X-ray powder diffraction pattern optionally has diffraction peaks at 2, 4, 6, 8 or 10 of the above-mentioned 2θ.

Preferably, the X-ray powder diffraction pattern thereof has at least a diffraction peak at one or more of 2θ of 9.2±0.2°, 10.2±0.2°, 16.6±0.2° or 18.4±0.2°, preferably at 2 to 4 of the above-mentioned 2θ, more preferably at 3 or 4 of the above-mentioned 2θ, and most preferably at 4 of the above-mentioned 2θ; optionally, further, the X-ray powder diffraction pattern can also have a diffraction peak at one or more of 2θ of 12.8±0.2°, 17.3±0.2°, 19.0±0.2°, 21.3±0.2° or 26.5±0.2°, preferably at 2, 3, 4 or 5 of the above-mentioned 2θ; for example, the X-ray powder diffraction pattern has diffraction peaks at 2θ of
9.2±0.2°, 10.2±0.2°, 16.6±0.2°, 18.4±0.2°;
9.2±0.2°, 10.2±0.2°, 16.6±0.2°, 17.3±0.2°;
9.2±0.2°, 12.8±0.2°, 16.6±0.2°, 18.4±0.2°;
9.2±0.2°, 10.2±0.2°, 17.3±0.2°, 19.0±0.2°;
9.2±0.2°, 12.8±0.2°, 17.3±0.2°, 19.0±0.2°;
10.2±0.2°, 16.6±0.2°, 17.3±0.2°, 21.3±0.2°;
10.2±0.2°, 12.8±0.2°, 16.6±0.2°, 17.3±0.2°;
12.8±0.2°, 17.3±0.2°, 19.0±0.2°, 21.3±0.2°;
9.2±0.2°, 10.2±0.2°, 16.6±0.2°, 18.4±0.2°; 19.0±0.2°;
9.2±0.2°, 10.2±0.2°, 16.6±0.2°, 17.3±0.2°; 19.0±0.2°;
9.2±0.2°, 12.8±0.2°, 16.6±0.2°, 18.4±0.2°; 19.0±0.2°;
9.2±0.2°, 10.2±0.2°, 17.3±0.2°, 19.0±0.2°; 21.3±0.2°;
9.2±0.2°, 12.8±0.2°, 17.3±0.2°, 19.0±0.2°; 21.3±0.2°;
10.2±0.2°, 16.6±0.2°, 17.3±0.2°, 21.3±0.2°; 26.5±0.2°;
10.2±0.2°, 12.8±0.2°, 16.6±0.2°, 17.3±0.2°; 19.0±0.2°;
12.8±0.2°, 17.3±0.2°, 19.0±0.2°, 21.3±0.2°; 26.5±0.2°;
9.2±0.2°, 10.2±0.2°, 16.6±0.2°, 18.4±0.2°; 19.0±0.2°; 21.3±0.2°;
9.2±0.2°, 10.2±0.2°, 16.6±0.2°, 17.3±0.2°; 19.0±0.2°; 21.3±0.2°;
9.2±0.2°, 12.8±0.2°, 16.6±0.2°, 18.4±0.2°; 19.0±0.2°; 21.3±0.2°;
9.2±0.2°, 10.2±0.2°, 17.3±0.2°, 19.0±0.2°; 21.3±0.2°; 26.5±0.2°;
9.2±0.2°, 12.8±0.2°, 17.3±0.2°, 19.0±0.2°; 21.3±0.2°; 26.5±0.2°;
10.2±0.2°, 16.6±0.2°, 17.3±0.2°, 18.4±0.2°; 21.3±0.2°; 26.5±0.2°;
10.2±0.2°, 12.8±0.2°, 16.6±0.2°, 17.3±0.2°; 18.4±0.2°; 19.0±0.2°; or
12.8±0.2°, 17.3±0.2°, 18.4±0.2°; 19.0±0.2°, 21.3±0.2°; 26.5±0.2°.

More preferably, the X-ray powder diffraction pattern thereof optionally also has a diffraction peak at one or more of 2θ of 8.6±0.2°, 10.8±0.2°, 12.0±0.2°, 13.7±0.2°, 14.2±0.2° or 28.6±0.2°, preferably at any 2 to 4, or any 5 or 6 of the above-mentioned 2θ, and further preferably at any 4 or 6 of the above-mentioned 2θ; for example, the X-ray powder diffraction pattern has diffraction peaks at 2θ of
8.6±0.2°, 10.8±0.2°, 12.0±0.2°, 13.7±0.2°;
8.6±0.2°, 10.8±0.2°, 12.0±0.2°, 14.2±0.2°;
8.6±0.2°, 10.8±0.2°, 14.2±0.2°, 28.6±0.2°;
10.8±0.2°, 12.0±0.2°, 13.7±0.2°, 14.2±0.2°;
12.0±0.2°, 13.7±0.2°, 14.2±0.2°, 28.6±0.2°;
8.6±0.2°, 10.8±0.2°, 12.0±0.2°, 13.7±0.2°, 14.2±0.2°;
8.6±0.2°, 10.8±0.2°, 12.0±0.2°, 14.2±0.2°, 28.6±0.2°; or
8.6±0.2°, 10.8±0.2°, 12.0±0.2°, 13.7±0.2°, 14.2±0.2°, 28.6±0.2°.

The X-ray powder diffraction pattern of the crystal form I has characteristic peaks at 2θ of 9.2±0.2° and 18.4±0.2°; preferably, the X-ray powder diffraction pattern also has characteristic peaks at 2θ of 10.2±0.2°, 16.6±0.2°, 17.3±0.2° and 19.0±0.2°; more preferably, the X-ray powder diffraction pattern also has characteristic peaks at 2θ of 12.8±0.2° and 21.3±0.2°; further preferably, the X-ray powder diffraction pattern also has characteristic peaks at 2θ of 26.5±0.2° and 28.6±0.2°, more further preferably, the X-ray powder diffraction pattern also has characteristic peaks at 8.6±0.2°, 10.8±0.2°, 12.0±0.2°, 13.7±0.2°, 14.2±0.2°, 15.5±0.2°, 19.5±0.2°, 20.5±0.2°, 23.1±0.2°, 27.8±0.2° and 32.1±0.2°.

By using Cu-Kα radiation, the X-ray characteristic diffraction peaks represented by 2θ angles and interplanar spacing d values are shown in Table 1.

**Table 1**

| Serial No. | XRPD data of free base crystal form I | | | |
|---|---|---|---|---|
| | 2θ (±0.2o) | d value | Peak height | Proportion (I%) |
| 1 | 8.637 | 10.2297 | 196 | 5.7 |
| 2 | 9.186 | 9.6187 | 3440 | 100.0 |
| 3 | 10.241 | 8.6309 | 536 | 15.6 |
| 4 | 10.814 | 8.1748 | 125 | 3.6 |
| 5 | 12.048 | 7.3401 | 93 | 2.7 |
| 6 | 12.815 | 6.9022 | 477 | 13.9 |
| 7 | 13.690 | 6.4630 | 130 | 3.8 |
| 8 | 14.176 | 6.2425 | 381 | 11.1 |
| 9 | 14.607 | 6.0594 | 76 | 2.2 |
| 10 | 15.474 | 5.7217 | 273 | 7.9 |
| 11 | 15.812 | 5.6002 | 29 | 0.8 |
| 12 | 16.592 | 5.3396 | 720 | 20.9 |
| 13 | 17.302 | 5.1210 | 627 | 18.2 |
| 14 | 18.417 | 4.8134 | 1820 | 52.9 |
| 15 | 19.045 | 4.6561 | 831 | 24.2 |
| 16 | 19.493 | 4.5501 | 117 | 3.4 |
| 17 | 20.173 | 4.3982 | 28 | 0.8 |
| 18 | 20.544 | 4.3197 | 282 | 8.2 |
| 19 | 21.331 | 4.1619 | 538 | 15.6 |
| 20 | 22.264 | 3.9896 | 46 | 1.3 |
| 21 | 23.119 | 3.8440 | 437 | 12.7 |
| 22 | 24.238 | 3.6690 | 46 | 1.3 |
| 23 | 25.185 | 3.5331 | 83 | 2.4 |
| 24 | 25.997 | 3.4246 | 76 | 2.2 |
| 25 | 26.482 | 3.3630 | 617 | 17.9 |
| 26 | 27.780 | 3.2087 | 304 | 8.8 |
| 27 | 28.570 | 3.1217 | 547 | 15.9 |
| 28 | 29.053 | 3.0709 | 32 | 0.9 |
| 29 | 29.447 | 3.0308 | 93 | 2.7 |
| 30 | 31.799 | 2.8117 | 32 | 0.9 |
| 31 | 32.098 | 2.7863 | 153 | 4.4 |
| 32 | 32.515 | 2.7514 | 38 | 1.1 |
| 33 | 34.127 | 2.6251 | 31 | 0.9 |
| 34 | 36.682 | 2.4479 | 26 | 0.8 |
| 35 | 37.474 | 2.3979 | 63 | 1.8 |
| 36 | 38.146 | 2.3572 | 27 | 0.8 |
| 37 | 38.747 | 2.3220 | 52 | 1.5 |
| 38 | 39.028 | 2.3060 | 50 | 1.5 |

The crystal form I of the compound 9 of the present invention has an X-ray powder diffraction pattern substantially as shown in FIG. 1, a DSC pattern substantially as shown in FIG. 2, and a TGA pattern substantially as shown in FIG. 3.

The crystal form I of the compound 9 of the present invention is an anhydrate crystal form.

The X-ray powder diffraction pattern of the crystal form II has a diffraction peak at 2θ of 9.9±0.2°, or has a diffraction peak at 2θ of 10.8±0.2°, or has a diffraction peak at 2θ of 13.2±0.2°, or has a diffraction peak at 2θ of 14.9±0.2°, or has a diffraction peak at 2θ of 16.4±0.2°, or has a diffraction peak at 2θ of 17.1±0.2°, or has a diffraction peak at 2θ of 17.9±0.2°, or has a diffraction peak at 2θ of 19.3±0.2°, or has a diffraction peak at 2θ of 19.8±0.2°, or has a diffraction peak at 2θ of 20.2±0.2°, or has a diffraction peak at 2θ of 20.5±0.2°, or has a diffraction peak at 2θ of 21.0±0.2°, or has a diffraction peak at 2θ of 21.9±0.2°, or has a diffraction peak at 2θ of 25.8±0.2°, or has a diffraction peak at 2θ of 26.5±0.2°, or has a diffraction peak at 2θ of 27.5±0.2°; preferably, the X-ray powder diffraction pattern optionally has diffraction peaks at 2, 4, 6, 8 or 10 of the above-mentioned 2θ.

Preferably, the X-ray powder diffraction pattern thereof has at least a diffraction peak at one or more of 2θ of 16.4±0.2°, 17.1±0.2°, 17.9±0.2° or 25.8±0.2°, preferably at 2 to 4 of the above-mentioned 2θ, more preferably at 3 or 4 of the above-mentioned 2θ, and most preferably at 4 of the above-mentioned 2θ; optionally, further, the X-ray powder diffraction pattern can also have a diffraction peak at one or more of 2θ of 10.8±0.2°, 14.9±0.2°, 19.3±0.2°, 21.9±0.2° or 26.5±0.2°, preferably at 2, 3, 4 or 5 of the above-mentioned 2θ; for example, the X-ray powder diffraction pattern has diffraction peaks at 2θ of
16.4±0.2°, 17.1±0.2°, 17.9±0.2°, 25.8±0.2°;
16.4±0.2°, 17.1±0.2°, 17.9±0.2°, 26.5±0.2°;
16.4±0.2°, 17.1±0.2°, 25.8±0.2°, 26.5±0.2°;
10.8±0.2°, 14.9±0.2°, 17.9±0.2°, 25.8±0.2°;
10.8±0.2°, 17.1±0.2°, 19.3±0.2°, 25.8±0.2°;
16.4±0.2°, 17.1±0.2°, 17.9±0.2°, 21.9±0.2°;
10.8±0.2°, 16.4±0.2°, 17.1±0.2°, 19.3±0.2°;
16.4±0.2°, 17.1±0.2°, 17.9±0.2°, 25.8±0.2°;
16.4±0.2°, 17.1±0.2°, 17.9±0.2°, 25.8±0.2°, 26.5±0.2°;
16.4±0.2°, 17.1±0.2°, 17.9±0.2°, 21.9±0.2°, 26.5±0.2°;
16.4±0.2°, 17.1±0.2°, 19.3±0.2°, 25.8±0.2°, 26.5±0.2°;
10.8±0.2°, 14.9±0.2°, 17.9±0.2°, 25.8±0.2°, 26.5±0.2°;
10.8±0.2°, 17.1±0.2°, 19.3±0.2°, 25.8±0.2°; 26.5±0.2°;
16.4±0.2°, 17.1±0.2°, 17.9±0.2°, 21.9±0.2°; 25.8±0.2°;
10.8±0.2°, 16.4±0.2°, 17.1±0.2°, 19.3±0.2°; 21.9±0.2°;
16.4±0.2°, 17.1±0.2°, 17.9±0.2°, 25.8±0.2°; 26.5±0.2°;
14.9±0.2°, 16.4±0.2°, 17.1±0.2°, 17.9±0.2°, 25.8±0.2°; 26.5±0.2°;
14.9±0.2°, 16.4±0.2°, 17.1±0.2°, 17.9±0.2°, 26.5±0.2°; 26.5±0.2°;
14.9±0.2°, 16.4±0.2°, 17.1±0.2°, 19.3±0.2°, 25.8±0.2°, 26.5±0.2°;
10.8±0.2°, 14.9±0.2°, 17.9±0.2°, 21.9±0.2°, 25.8±0.2°; 26.5±0.2°;
10.8±0.2°, 17.1±0.2°, 19.3±0.2°, 21.9±0.2°, 25.8±0.2°; 26.5±0.2°;
16.4±0.2°, 17.1±0.2°, 17.9±0.2°, 21.9±0.2°; 25.8±0.2°; 26.5±0.2°;
10.8±0.2°, 14.9±0.2°, 16.4±0.2°, 17.1±0.2°, 19.3±0.2°; 21.9±0.2°; or
10.8±0.2°, 16.4±0.2°, 17.1±0.2°, 17.9±0.2°, 25.8±0.2°; 26.5±0.2°.

More preferably, the X-ray powder diffraction pattern thereof optionally also has a diffraction peak at one or more of 2θ of 9.9±0.2°, 13.2±0.2°, 19.8±0.2°, 20.2±0.2°, 20.5±0.2° or 21.0±0.2°, preferably at any 2 to 4, or any 5 or 6 of the above-mentioned 2θ, and further preferably at any 4 or 6 of the above-mentioned 2θ; for example, the X-ray powder diffraction pattern has diffraction peaks at 2θ of
9.9±0.2°, 13.2±0.2°, 19.8±0.2°, 20.2±0.2°,
9.9±0.2°, 13.2±0.2°, 19.8±0.2°, 20.5±0.2°,
9.9±0.2°, 13.2±0.2°, 20.5±0.2°, 21.0±0.2°,
13.2±0.2°, 19.8±0.2°, 20.2±0.2°, 20.5±0.2°,
9.9±0.2°, 13.2±0.2°, 19.8±0.2°, 20.2±0.2°, 20.5±0.2°,
13.2±0.2°, 19.8±0.2°, 20.2±0.2°, 20.5±0.2°, 21.0±0.2°; or
9.9±0.2°, 13.2±0.2°, 19.8±0.2°, 20.2±0.2°, 20.5±0.2° or 21.0±0.2°.

In a further preferred embodiment of the present invention, the X-ray powder diffraction pattern of the crystal form II has characteristic peaks at 2θ of 16.4±0.2° and 25.8±0.2°; preferably, the X-ray powder diffraction pattern also has characteristic peaks at 2θ of 17.1±0.2°, 17.9±0.2°, 21.9±0.2° and 26.5±0.2°; more preferably, the X-ray powder diffraction pattern also has characteristic peaks at 2θ of 10.8±0.2° and 14.9±0.2°; further preferably, the X-ray powder diffraction pattern also has characteristic peaks at 2θ of 9.9±0.2° and 19.3±0.2°, more further preferably, the X-ray powder diffraction pattern also has characteristic peaks at 13.2±0.2°, 19.8±0.2°, 20.2±0.2°, 20.5±0.2°, 21.0±0.2° and 27.5±0.2°.

By using Cu-Kα radiation, the X-ray characteristic diffraction peaks represented by 2θ angles and interplanar spacing d values are shown in Table 2.

**Table 2**

| Serial No. | XRPD data of free base crystal form II | | | |
|---|---|---|---|---|
| | 2θ (±0.2o) | d value | Peak height | Proportion (I%) |
| 1 | 8.909 | 9.9176 | 938 | 23.8 |
| 2 | 9.638 | 9.1690 | 110 | 2.8 |
| 3 | 9.861 | 8.9618 | 374 | 9.5 |
| 4 | 10.771 | 8.2069 | 436 | 11.1 |
| 5 | 12.820 | 6.8996 | 50 | 1.3 |
| 6 | 13.208 | 6.6976 | 243 | 6.2 |
| 7 | 14.621 | 6.0533 | 149 | 3.8 |
| 8 | 14.892 | 5.9440 | 581 | 14.7 |
| 9 | 15.898 | 5.5698 | 192 | 4.9 |
| 10 | 16.386 | 5.4052 | 3941 | 100.0 |
| 11 | 17.137 | 5.1699 | 605 | 15.4 |
| 12 | 17.464 | 5.0738 | 44 | 1.1 |
| 13 | 17.854 | 4.9639 | 738 | 18.7 |
| 14 | 18.346 | 4.8319 | 61 | 1.5 |
| 15 | 18.518 | 4.7875 | 139 | 3.5 |
| 16 | 19.327 | 4.5888 | 481 | 12.2 |
| 17 | 19.795 | 4.4813 | 279 | 7.1 |
| 18 | 20.237 | 4.3844 | 210 | 5.3 |
| 19 | 20.466 | 4.3360 | 396 | 10.0 |
| 20 | 20.990 | 4.2288 | 452 | 11.5 |
| 21 | 21.620 | 4.1069 | 42 | 1.1 |
| 22 | 21.888 | 4.0573 | 571 | 14.5 |
| 23 | 22.471 | 3.9533 | 67 | 1.7 |
| 24 | 22.670 | 3.9191 | 176 | 4.5 |
| 25 | 23.035 | 3.8578 | 68 | 1.7 |
| 26 | 23.220 | 3.8274 | 93 | 2.4 |
| 27 | 24.014 | 3.7028 | 83 | 2.1 |
| 28 | 24.283 | 3.6622 | 67 | 1.7 |
| 29 | 24.821 | 3.5841 | 92 | 2.3 |
| 30 | 24.983 | 3.5612 | 118 | 3.0 |
| 31 | 25.794 | 3.4511 | 1621 | 41.1 |
| 32 | 26.284 | 3.3879 | 360 | 9.1 |
| 33 | 26.483 | 3.3629 | 748 | 19.0 |
| 34 | 26.925 | 3.3086 | 174 | 4.4 |
| 35 | 27.538 | 3.2364 | 265 | 6.7 |
| 36 | 28.469 | 3.1327 | 140 | 3.6 |
| 37 | 29.118 | 3.0643 | 50 | 1.3 |
| 38 | 29.466 | 3.0288 | 64 | 1.6 |
| 39 | 30.162 | 2.9605 | 52 | 1.3 |
| 40 | 30.826 | 2.8982 | 41 | 1.0 |
| 41 | 31.347 | 2.8513 | 39 | 1.0 |
| 42 | 32.343 | 2.7657 | 94 | 2.4 |
| 43 | 32.745 | 2.7326 | 84 | 2.1 |
| 44 | 33.804 | 2.6494 | 192 | 4.9 |
| 45 | 34.834 | 2.5734 | 47 | 1.2 |
| 46 | 36.664 | 2.4491 | 31 | 0.8 |
| 47 | 37.187 | 2.4158 | 69 | 1.8 |
| 48 | 38.376 | 2.3436 | 72 | 1.8 |
| 49 | 38.976 | 2.3089 | 65 | 1.6 |
| 50 | 39.397 | 2.2852 | 81 | 2.1 |

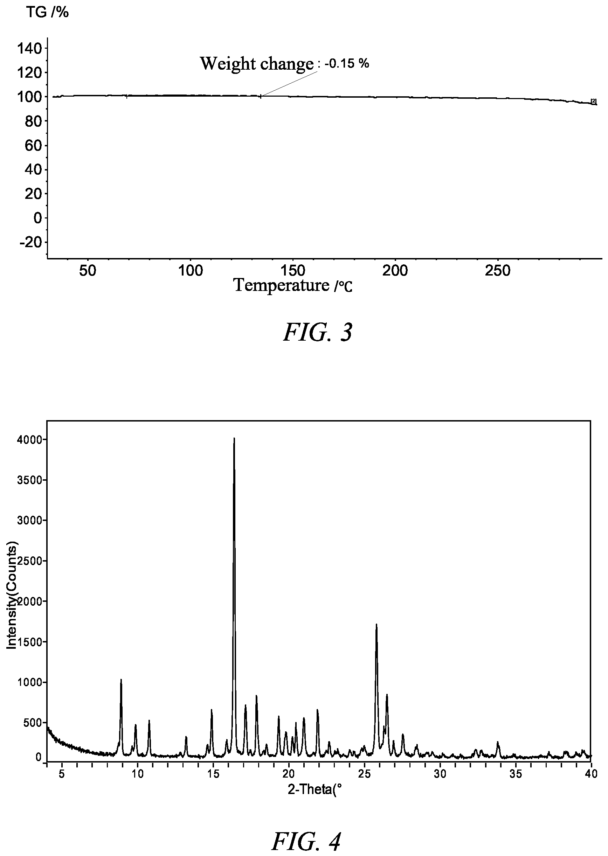

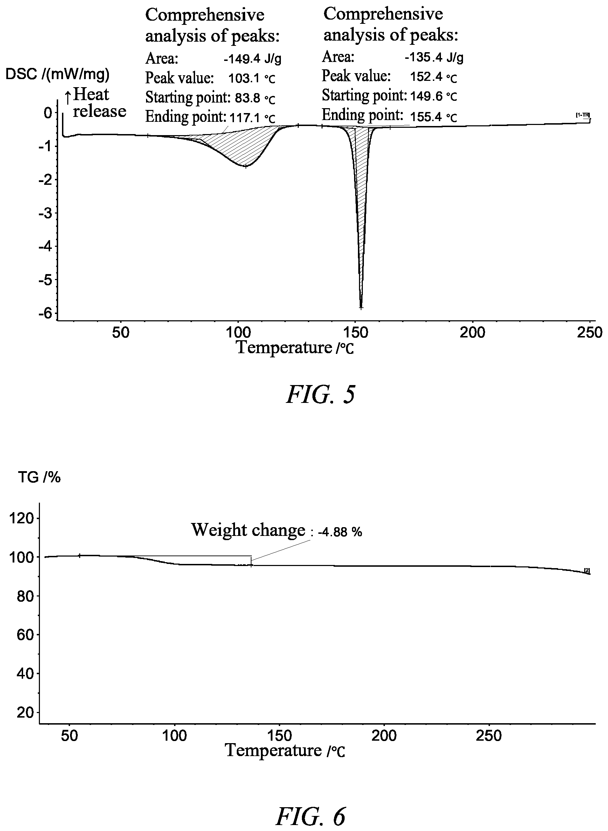

The crystal form II of the compound 9 of the present invention has an X-ray powder diffraction pattern substantially as shown in FIG. 4, a DSC pattern substantially as shown in FIG. 5, and a TGA pattern substantially as shown in FIG. 6.

The water content of the crystal form II (monohydrate) of the compound 9 of the present invention measured by KF is 3.1% (API : water = 1 : 0.83, molar ratio).

The present invention also provides a method for preparing the crystal form of the compound represented by general formula (I), wherein the method specifically comprises the following steps:
1) weighing an appropriate amount of a free base, and suspending the free base with a poor solvent;
2) shaking a suspension obtained above;
3) quickly centrifuging the suspension, removing a supernatant, and drying a solid to a constant weight to afford a target product;
wherein
the poor solvent is selected from acetone, ethyl acetate, isopropyl acetate, acetonitrile, ethanol, tetrahydrofuran, 2-methyltetrahydrofuran, dichloromethane, 1,4-dioxane, benzene, toluene, isopropanol, n-butanol, isobutanol, N,N-dimethylformamide, N,N-dimethylacetamide, n-propanol, tert-butanol, 2-butanone or 3-pentanone, methyl tert-butyl ether, water, heptane or n-pentane; preferably isopropyl acetate, toluene, methyl tert-butyl ether, water, heptane or n-pentane.

In a preferred embodiment of the present invention, the suspension density in step 1) is 50-200 mg/mL.

In a preferred embodiment of the present invention, the temperature in step 2) is 0°C-50°C.

In a preferred embodiment of the present invention, the shaking time in step 2) is selected from 1-10 days.

The present invention further provides a method for preparing the crystal form of the compound represented by general formula (I), wherein the method specifically comprises the following steps:
1) weighing an appropriate amount of a free base, and dissolving the free base with a good solvent;
2) adding an anti-solvent to a solution obtained above and performing stirring until a solid precipitates;
3) quickly centrifuging a suspension obtained above, removing a supernatant, and drying the remaining solid to a constant weight to afford a target product;
wherein
the good solvent is selected from methanol, acetone, ethyl acetate, acetonitrile, ethanol, tetrahydrofuran, dichloromethane, 1,4-dioxane, benzene, toluene, isopropanol, n-butanol, isobutanol, N,N-dimethylformamide, N,N-dimethylacetamide, n-propanol, tert-butanol, 2-butanone or 3-pentanone; preferably methanol, dichloromethane or ethyl acetate.

The anti-solvent is selected from heptane, water, methyl tert-butyl ether, cyclohexane or isopropyl acetate.

In a preferred embodiment of the present invention, the temperature in step 2) is 0°C-25°C.

The present invention further provides a method for preparing the crystal form of the compound represented by general formula (I), wherein the method specifically comprises the following steps:
1) weighing an appropriate amount of a free base, and heating and dissolving the free base with a good solvent;
2) quickly placing a solution obtained above under low temperature and stirring the solution until a solid precipitates;
3) quickly centrifuging a suspension obtained above, removing a supernatant, placing a remaining solid in a drying oven and drying to a constant weight to afford a target product;
wherein
the good solvent is selected from methanol, acetone, ethyl acetate, acetonitrile, ethanol, tetrahydrofuran, dichloromethane, 1,4-dioxane, benzene, toluene, isopropanol, n-butanol, isobutanol, N,N-dimethylformamide, N,N-dimethylacetamide, n-propanol, tert-butanol, 2-butanone or tetrahydrofuran; preferably acetone.

In a preferred embodiment of the present invention, the temperature in step 2) is 0°C-25°C.

Another objective of the present invention is to provide a pharmaceutical composition comprising a therapeutically-effective amount of the above-mentioned crystal form of the compound, and one or more pharmaceutically acceptable carriers, diluents or excipients.

The present invention further relates to the use of the crystal form of any compound or the pharmaceutical composition of the present invention in the preparation of an orexin receptor antagonist; preferably the use of the crystal form or the pharmaceutical composition in the preparation of an OX2R selective receptor antagonist.

The present invention further relates to the use of the crystal form of any compound or the pharmaceutical composition of the present invention in the preparation of a drug for treating nervous system disease; wherein the nervous system disease is preferably insomnia, depression, anxiety or drug addiction, more preferably major depressive disorder (MDD), primary and secondary insomnia or depression accompanied by insomnia.

The present invention further relates to a method for treating nervous system diseases, wherein the crystal form of any compound further involved in the present invention or the pharmaceutical composition of the present invention is used; wherein the nervous system disease is preferably insomnia, depression, anxiety or drug addiction, more preferably major depressive disorder (MDD), primary and secondary insomnia or depression accompanied by insomnia.

### Brief Description of the Drawings

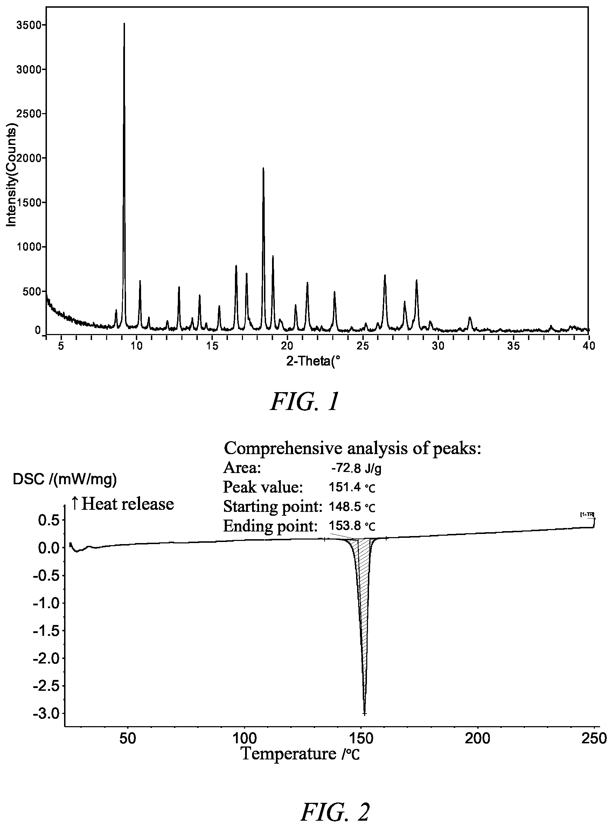

FIG. 1 is an XRPD pattern of the free base crystal form I of compound 9.
FIG. 2 is a DSC pattern of the free base crystal form I of compound 9.
FIG. 3 is a TGA pattern of the free base crystal form I of compound 9.
FIG. 4 is a XRPD pattern of the free base crystal form II of compound 9.
FIG. 5 is a DSC pattern of the free base crystal form II of compound 9.
FIG. 6 is a TGA pattern of the free base crystal form II of compound 9.

### Detailed Description of Embodiments

Unless stated to the contrary, the terms used in the description and claims have the following meanings.

In the present invention, alkyl refers to a saturated aliphatic hydrocarbon group, which is a straight or branched group containing 1 to 20 carbon atoms, preferably alkyl containing 1 to 8 carbon atoms, more preferably alkyl containing 1 to 6 carbon atoms, and most preferably alkyl containing 1 to 3 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and various branched isomers thereof, etc.

Alkyl can be substituted or unsubstituted. When the alkyl is substituted, the substituent can be substituted at any available connection point, and the substituent is preferably one or more of groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, oxo, carboxyl or a carboxylate group. In the present invention, alkyl is preferably methyl, ethyl, isopropyl, tert-butyl, haloalkyl, deuteroalkyl, alkoxy-substituted alkyl and hydroxyl-substituted alkyl; and the hydroxyl-substituted alkyl can be 2-hydroxyisopropyl or 1-hydroxyethyl.

In the present invention, the cycloalkyl refers to a saturated or partially unsaturated monocyclic or polycyclic cyclic hydrocarbon substituent, and the cycloalkyl ring comprises 3 to 20 carbon atoms, preferably 3 to 12 carbon atoms, more preferably 3 to 8 carbon atoms, and further preferably 3 to 6 carbon atoms.

Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, etc.; and the polycyclic cycloalkyl includes spirocycloalkyl, fused cycloalkyl and bridged cycloalkyl.

The cycloalkyl ring can be fused to an aryl, heteroaryl or heterocycloalkyl ring, wherein the ring connected to the parent structure is cycloalkyl, and non-limiting examples include indanyl, tetrahydronaphthyl, benzocycloheptanyl, etc. Cycloalkyl can be optionally substituted or unsubstituted. When the cycloalkyl is substituted, the substituent is preferably one or more of groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, oxo, carboxyl or a carboxylate group.

In the present invention, heterocyclyl refers to a saturated or partially unsaturated monocyclic or polycyclic heterocyclyl, which comprises 3 to 20 ring atoms, wherein one or more ring atoms are heteroatoms selected from nitrogen, oxygen or S(O)ₘ (wherein m is an integer of 0 to 2), but excluding ring moieties of -O-O-, -O-S- or -S-S-, and the remaining ring atoms are carbon. Preferably, the heterocyclyl comprises 3 to 12 ring atoms, of which 1-4 are heteroatoms; more preferably, the heterocyclyl comprises 3 to 10 ring atoms; and further preferably, the heterocyclyl comprises 3 to 8 ring atoms. Non-limiting examples of monocyclic heterocyclyl include pyrrolidinyl, pyrrolidonyl, piperidin-2-onyl, 3,4-dihydropyridin-2(1H)-onyl, 4,5-dihydropyridazin-3(2H)-onyl, azetidinyl, oxetanyl, oxacyclohexyl, imidazolidinyl, tetrahydrofuryl, tetrahydrothienyl, dihydroimidazolyl, dihydrofuryl, dihydropyrazolyl, dihydropyrrolyl, piperidyl, piperazinyl, morpholinyl, thiomorpholinyl, homopiperazinyl, pyranyl, 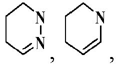 etc.; preferably pyrrolidinyl, pyrrolidonyl, piperidin-2-onyl, 3,4-dihydropyridin-2(1H)-onyl, 4,5-dihydropyridazin-3(2H)-onyl, azetidinyl, oxetanyl, dihydropyrrolyl, tetrahydrofuryl, pyrazolidinyl, morpholinyl, 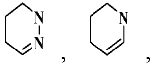 piperazinyl and pyranyl; and more preferably dihydropyrrolyl, pyrrolidinyl, pyrrolidonyl, piperidin-2-onyl, 3,4-dihydropyridin-2(1H)-onyl, 4,5-dihydropyridazin-3(2H)-onyl, azetidinyl, oxetanyl, oxacyclohexyl, morpholinyl, piperidyl, piperazinyl, 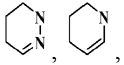 or pyranyl. Polycyclic heterocyclyl includes spiro, fused and bridged heterocyclyl, wherein the spiro, fused and bridged heterocyclyl groups are optionally connected to other groups through a single bond, or further fused to other cycloalkyl, heterocyclyl, aryl and heteroaryl through any two or more atoms on the ring.

Heterocyclyl can be optionally substituted or unsubstituted. When the heterocyclyl is substituted, the substituent is preferably one or more of groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, oxo, carboxyl or a carboxylate group.

**In** the present invention, aryl refers to a 6- to 14-membered all-carbon monocyclic or fused polycyclic (*i.e.*, rings sharing adjacent pairs of carbon atoms) group having a conjugated π electron system, preferably 6- to 10-membered aryl, more preferably 6- to 8-membered aryl, such as phenyl and naphthyl, preferably phenyl. The aryl ring can be fused to a heteroaryl, heterocyclyl or cycloalkyl ring, wherein the ring connected to the parent structure is an aryl ring, and the non-limiting examples thereof include:

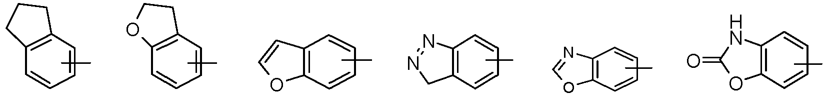

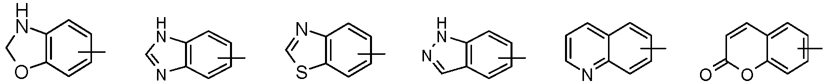

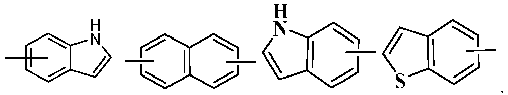

Aryl can be substituted or unsubstituted. When the aryl is substituted, the substituent is preferably one or more of groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl or a carboxylate group.

In the present invention, heteroaryl refers to a heteroaromatic system containing 1 to 4 heteroatoms and 5 to 14 ring atoms, wherein the heteroatoms are selected from oxygen, sulphur and nitrogen. The heteroaryl is preferably 5- to 10-membered, more preferably 5- to 8-membered, and most preferably 5-membered or 6-membered, e.g., pyrazinyl, pyridazinyl, imidazolyl, furyl, thienyl, thiazolyl, pyrazolyl, oxazolyl, isoxazolyl, pyrrolyl, triazolyl, tetrazolyl, pyridyl, pyrimidyl, thiadiazole, oxadiazole, pyrazinyl, etc., preferably pyrimidyl, pyrazolyl, oxazolyl, isoxazolyl, oxadiazole, or pyridine. The heteroaryl ring can be fused to an aryl, heterocyclyl or cycloalkyl ring, wherein the ring connected to the parent structure is heteroaryl ring, and the non-limiting examples thereof include:

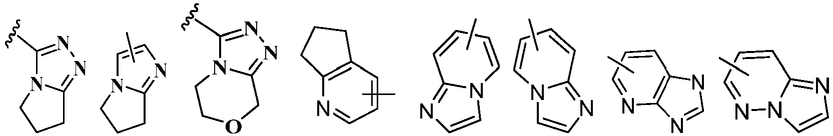

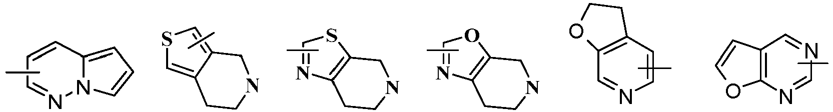

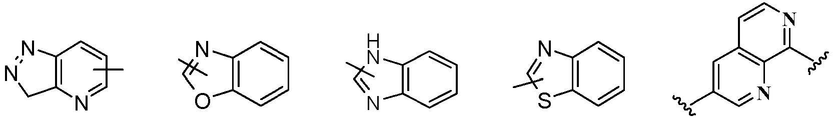

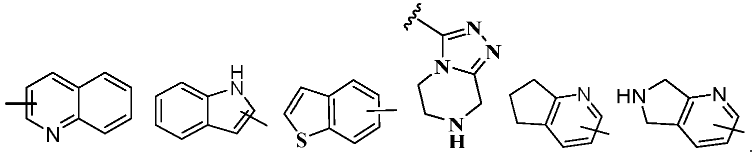

Heteroaryl can be optionally substituted or unsubstituted. When the heteroaryl is substituted, the substituent is preferably one or more of groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl or a carboxylate group.

In the present invention, alkoxy refers to -O-(alkyl) and -O-(unsubstituted cycloalkyl), wherein alkyl is as defined above, preferably alkyl containing 1 to 8 carbon atoms, more preferably alkyl containing 1 to 6 carbon atoms, and most preferably alkyl containing 1 to 3 carbon atoms. Non-limiting examples of alkoxy include: methoxy, ethoxy, propoxy, butoxy, cyclopropoxy, cyclobutoxy, cyclopentoxy, and cyclohexoxy. Alkoxy can be optionally substituted or unsubstituted. When the alkoxy is substituted, the substituent is preferably one or more of groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl or a carboxylate group.

Non-limiting examples of alkoxy also include: prop-2-oxy, etc.

In the present invention, "haloalkyl" refers to alkyl substituted with one or more halogen, wherein alkyl is defined as above. Non-limiting examples of haloalkyl include: trifluoromethyl and trifluoroethyl.

Non-limiting examples of haloalkyl also include: difluoromethyl, 1,1,2,2-tetrafluoroethyl, perfluoroethyl, etc.

In the present invention, haloalkoxy refers to alkoxy substituted with one or more halogen, wherein alkoxy is defined as above.

The haloalkoxy can be fully halogenated or partially halogenated, and the number of halo can be 1, 2, 3, 4, 5, 6, 7, 8, 9, 10, etc. Halogen is preferably F, Cl, Br, or I. For example, haloalkoxy can be trifluoromethoxy, difluoromethoxy, 1,1,2,2-tetrafluoroethoxy, perfluoroethoxy, etc.

In the present invention, hydroxyalkyl refers to alkyl substituted with hydroxyl, wherein alkyl is defined as above.

In the present invention, alkenyl refers to a chain alkenyl group, also known as an alkene group, preferably alkenyl containing 2 to 8 carbon atoms, more preferably alkenyl containing 2 to 6 carbon atoms, more further preferably alkenyl containing 2 to 4 carbon atoms, and most preferably alkenyl containing 2 or 3 carbon atoms. Non-limiting examples of alkenyl include: ethenyl and propenyl. Moreover, the alkenyl can be further substituted with other related groups, such as: alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl or a carboxylate group.

In the present invention, alkynyl refers to a chain alkynyl group, also known as an alkyne group, and means an unsaturated hydrocarbon group containing - C≡C-, preferably alkynyl containing 2 to 8 carbon atoms, more preferably alkynyl containing 2 to 6 carbon atoms, more further preferably alkynyl containing 2 to 4 carbon atoms, and most more preferably alkynyl containing 2 or 3 carbon atoms. Moreover, the alkynyl can be further substituted with other related groups, such as: alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl or a carboxylate group.

In the present invention, haloalkyl refers to alkyl substituted with one or more halogen, wherein alkyl is defined as above.

In the present invention, haloalkoxy refers to alkoxy substituted with one or more halogen, wherein alkoxy is defined as above.

In the present invention, hydroxyalkyl refers to alkyl substituted with hydroxyl, wherein alkyl is defined as above.

"Hydroxyl" refers to an -OH group.

"Halogen" refers to fluorine, chlorine, bromine or iodine.

"Amino" refers to -NH₂.

"Cyano" refers to -CN.

"Nitro" refers to -NO₂.

"THF" refers to tetrahydrofuran.

"DMSO" refers to dimethyl sulfoxide.

"IPA" refers to isopropanol.

"MeOH" refers to methanol.

"EtOH" refers to ethanol.

"DMF" refers to N, N-dimethylformamide.

"DIPEA" refers to N,N-diisopropylethylamine.

"HEPES" refers to 4-hydroxyethylpiperazine ethanesulfonic acid.

Different terms such as "X is selected from A, B, or C", "X is selected from A, B and C", "X is A, B or C", and "X is A, B and C" all express the same meaning, i.e., X can be any one or more of A, B, and C.

"Optional" or "optionally" means that the event or circumstance subsequently described may but need not to occur, and the description includes the occasion where the event or circumstance occurs or does not occur.

"Substituted" means that one or more hydrogen atoms, preferably at most 5, more preferably 1-3 hydrogen atoms in the group are each independently substituted with a corresponding number of substituents. It goes without saying, the substituents may be only in their possible chemical positions, a person skilled in the art can determine the possible or impossible substitutions (by experiment or theory) without paying too much effort. For example, the amino or a hydroxyl having free hydrogen may be unstable when combined the carbon atoms having an unsaturated (e.g., olefinic) bond.

"Stereoisomerism" comprises three types, geometric isomerism (cis-trans isomerism), optical isomerism, and conformational isomerism.

The hydrogen atoms of the present invention can be replaced with its isotope deuterium, and any hydrogen atom in the example compounds involved in the present invention can also be replaced with a deuterium atom.

"Pharmaceutical composition" denotes a mixture containing one or more compounds described herein or physiologically/pharmaceutically acceptable salts or prodrug thereof and other chemical components, as well as other components, such as physiologically/pharmaceutically acceptable carriers and excipients. The purpose of pharmaceutical compositions is to facilitate administration to living organisms and facilitate the absorption of active ingredients to exert biological activity.

X-ray powder diffraction pattern (XRPD) refers to the experimentally observed diffraction pattern or the parameters derived therefrom. The X-ray powder diffraction pattern is characterized by the peak position (abscissa) and the peak intensity (ordinate). Those skilled in the art will appreciate that the experimental error depends on the conditions of the instrument, the preparation of the sample and the purity of the sample. In particular, it is well known to those skilled in the art that the X-ray diffraction pattern often changes with the conditions of the instrument, and those skilled in the art should understand that the appropriate error tolerance of XRPD can be: 2θ±0.5°; 2θ±0.4°; 2θ±0.3°; or 2θ±0.2°. In particular, it should be noted that the relative intensity of the X-ray diffraction pattern may also change with experimental conditions, and thus the order of peak intensity cannot be used as the only or decisive factor. In addition, due to the influence of experimental factors such as sample height, peak angles may shift as a whole, and a certain shift is usually allowed. Therefore, those skilled in the art will appreciate that any crystal form having the characteristic peak identical or similar to those of the pattern of the present invention falls within the scope of the present invention.

"TGA" refers to thermogravimetric analysis (TGA) experiment.

"DSC" refers to differential scanning calorimetry (DSC) experiment.

"HPLC" refers to high performance liquid chromatography (HPLC) experiment.

"PK" refers to pharmacokinetic (PK) experiment.

"KF" refers to Karl Fischer moisture measurement (KF) experiment.

The present invention will be further described below in conjunction with examples, but these examples are not meant to limit the scope of the present invention.

### Example

### Preparation of intermediate:

### Preparation of intermediate 1: 2-(2-chloro-6-methylpyrimidin-4-yl)propanol

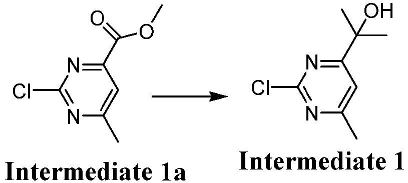

A solution of intermediate 1a (674 mg, 3.6 mmol) in THF (5 mL) was added to methylmagnesium bromide (5 mL, 1 M in THF) at -78°C, and the mixture was stirred at -78°C for 2 hours, and then stirred at room temperature for 12 hours. The reaction liquid was quenched by slowly adding saturated ammonium chloride solution (20 mL) and extracted with dichloromethane (20 mL*3), the organic phases were combined, dried and concentrated, and the residue was purified by silica gel column chromatography (petroleum ether/ethyl acetate system) to afford intermediate 1 (362 mg, colourless oil, 53.7% yield).
MS m/z (ESI): 187.1 [M+1]

### Preparation of intermediate 2: 2-(5-fluoro-2-(3aR,6 aS)-hexahydropyrrolo[3,4-c ]pyrrol-2(1H)-yl)-6-methylpyrimidin-4- yl)-propan-2-ol

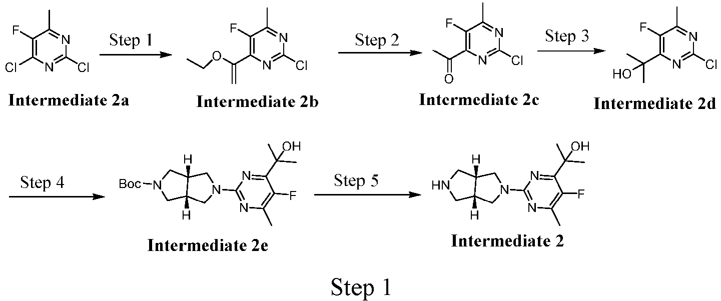

Under nitrogen protection, to a solution of intermediate 2a (1.3 g, 7.18 mmol) in anhydrous DMF (15 mL) were added tributyl-(1-ethoxy-ethenyl)-stannane (2.7 mL, 7.99 mmol) and bis(triphenylphosphine)palladium(II) dichloride (100 mg, 0.142 mmol). The mixture was heated at 100°C for 16 hours and cooled, saturated solution of potassium fluoride (aq) was added, and the mixture was stirred at room temperature for 1 hour. The mixture was filtered through celite, and the organic phase was washed thoroughly with water, then extracted with ethyl acetate, and concentrated. The crude product was subjected to column chromatography (PE : EA = 10 : 1) to obtain intermediate 2b (1.3 g, 83.5%).
MS m/z (ESI): 217.0 [M+1]

### Step 2

Intermediate 2b (1.3 g, 6.00 mmol) was dissolved in THF (10 mL), and 3N HCl (5 mL) was added. The mixture was stirred at room temperature for 1 hour. After the reaction was completed as monitored by LCMS, the reaction was adjusted to pH about 7-8 with saturated NaHCO₃ and extracted with ethyl acetate, and the organic phase was concentrated to afford intermediate 2c (1.1 g, 97.2%), which was directly used for the next step.
MS m/z (ESI): 189.0 [M+1]
1H NMR (400 MHz, Chloroform-d) δ 2.69 (s, 3H), 2.62 (d, J = 2.8 Hz, 3H).

### Step 3

Intermediate 2c (1.9 g, 10.07 mmol) and MeMgBr (3 M, 4.37 mL) were added to THF (40 mL) at 0°C. The reaction solution was stirred at 25°C for 1 hour, saturated NH₄Cl (20 ml) was added, and the mixture was extracted with ethyl acetate (40 ml). The combined extracts were dried over Na₂SO₄ and spun to dryness, and the residue was subjected to column chromatography (PE : EA = 3 : 1) to afford intermediate 2d (1.4 g, 67.9% yield).
MS m/z (ESI): 205.0[M+1]

### Step 4

Rac-tert-butyl (3aR,6aS)-2,3,3a,4,6,6a-hexahydro-1H-pyrrolo[3,4-c]pyrrole-5-carboxylate (500 mg, 2.36 mmol), intermediate 2d (530.16 mg, 2.59 mmol) and caesium carbonate (1.55 g, 4.76 mmol) were added to DMF (10 mL). The reaction solution was stirred at 100°C for 3 hours, water (20 mL) was added, and the mixture was extracted with dichloromethane (30 mL*2). The combined extracts were dried over Na₂SO₄ and spun to dryness, and the crude product was subjected to column chromatography (PE : EA = 3 : 1) to afford intermediate 2e (720 mg, 80.3% yield).
MS m/z (ESI): 381.2 [M+1]

### Step 5

Intermediate 2e (1 g, 2.63 mmol) and trifluoroacetic acid (2.69 mmol, 2 mL) were added to DCM (3 mL). The reaction solution was stirred at 25°C for 3 hours and spun to dryness to afford intermediate 2 (700 mg, 95.0% yield).
MS m/z (ESI): 281.2 [M+1]

### Preparation of intermediate 3: 2-(6-(3aR,6aS)-hexahydropyrrolo[3,4-c]pyrrol-2(1h)-yl)-4-methylpyridin-2-yl)propan-2-ol

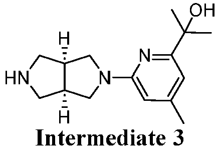

With reference to the preparation method of step 3, step 4 and step 5 of intermediate 2, intermediate 3 was prepared with 6-chloro-4-methyl -2-acetylpyridine instead of intermediate 2c, to afford intermediate 3 (1.2 g, colourless oil, 53.7%).
MS m/z (ESI): 262.2 [M+1]

### Example 1

### Preparation of compound 1: (2-fluoro-6-(2H-1,2,3-triazol-2-yl)phenyl)((3aR,6aS)-5-(4-(2-hydroxypropan-2-yl)-6-methylpyrimidin-2-yl)hexahydropyrrolo[3,4-c]pyrrol-2(1H)-yl)methanone

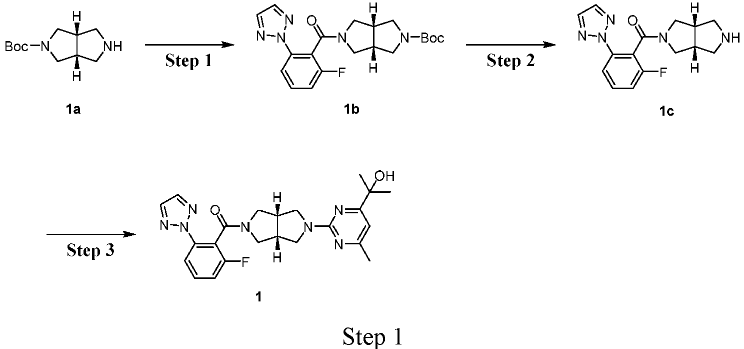

To a solution of 1a (300 mg, 1.42 mmol) and 2-fluoro-6-(2H-1,2,3-triazol-2-yl)benzoic acid (310 mg, 1.50 mmol) in DMF (5.0 mL) was slowly added HATU (809 mg, 2.13 mmol) and DIPEA (550 mg, 4.26 mmol), and the mixture was stirred at room temperature for 2 hours. The reaction liquid was diluted with ethyl acetate (30 mL) and washed with water (30 mL*3), the organic phases were combined, dried and concentrated, and the residue was purified by silica gel column chromatography (petroleum ether/ethyl acetate system) to afford tert-butyl (3aR,6aS)-5-(2-fluoro-6-(2H-1,2,3-triazol-2-yl)benzoyl)hexahydropyrrolo[3,4-c]pyrrole-2(1H)-carboxylate (compound 1b) (452 mg, light yellow solid, 79.7%).
MS m/z (ESI): 402.1 [M+1]

### Step 2

To a solution of 1b (450 mg, 1.12 mmol) in DCM (6.0 mL) was slowly added TFA (3.0 mL) at 0°C, and the mixture was stirred at room temperature for 2 hours. The reaction liquid was concentrated to afford (2-fluoro-6-(2H-1,2,3-triazol-2-yl)phenyl)((3 aR,6aS)-hexahydropyrrolo[3,4-c]pyrrol-2(1H)-yl)methanone (compound 1c) (330 mg, light yellow oil), which was directly used in the next step.
MS m/z (ESI): 302.0 [M+1]

### Step 3

To a round bottom flask were added intermediate 1 (61.5 mg, 0.33 mmol), 1c (100 mg, 0.33 mmol), Pd₂(dba)₃ (18 mg, 0.02 mmol), BINAP (25 mg, 0.04 mmol), caesium carbonate (215 mg, 0.66 mmol) and dioxane (2 mL), and the mixture was stirred at 100°C under nitrogen protection for 12 hours. The reaction liquid was cooled, quenched with water (5 mL) and extracted with dichloromethane (10 mL*3), the organic phases were combined, dried and concentrated, and the residue was purified by preparative HPLC, to afford (2-fluoro-6-(2H-1,2,3-triazol-2-yl)phenyl)((3aR,6aS)-5-(4-(2-hydroxypropan-2-yl)-6-methylpyrimidin-2-yl)hexahydropyrrolo[3,4-c]pyrrol-2(1H)-yl)methanone (compound 1) (32.4 mg, white solid, 21.8%).
MS m/z (ESI): 452.2 [M+1]
¹H NMR (400 MHz, DMSO-d6) δ 8.16 (s, 1H), 7.94 (s, 1H), 7.80 (dd, J = 17.2, 8.2 Hz, 1H), 7.66 (td, J = 8.3, 6.1 Hz, 1H), 7.44 (dt, J = 12.0, 8.6 Hz, 1H), 6.74 (d, J = 4.1 Hz, 1H), 5.10 (d, J = 4.0 Hz, 1H), 3.73 (dtd, J = 22.4, 7.2, 6.5, 3.6 Hz, 2H), 3.59 - 3.41 (m, 4H), 3.35 (d, J = 4.8 Hz, 1H), 3.16 - 2.90 (m, 3H), 2.28 (d, J = 3.5 Hz, 3H), 1.36 (d, J = 3.3 Hz, 6H).

### Example 2

### Preparation of compound 2: ((3aR,6aS)-5-(5-fluoro-4-(2-hydroxypropan-2-yl)-6-methylpyrimidin-2-yl)hexahydropyrrolo[3,4-c]pyrrol-2(1H)-yl )(2-fluoro-6-(2H-1,2,3-triazol-2-yl)phenyl)methanone

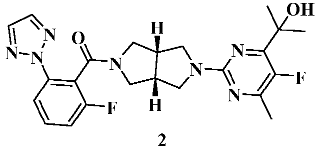

The synthesis method of compound 2 referred to the synthesis method of Example 1.
MS m/z (ESI): 470.2 [M+1].

### Example 3

### Preparation of compound 3: (2-fluoro-6-(pyrimidin-2-yl)phenyl)((3aR,6aS)-5-(6-(2-hydroxypropan-2-yl)-4-methylpyridin-2-yl)hexahydropyrrolo[3,4-c]pyrrol-2(1H)-yl)methanone

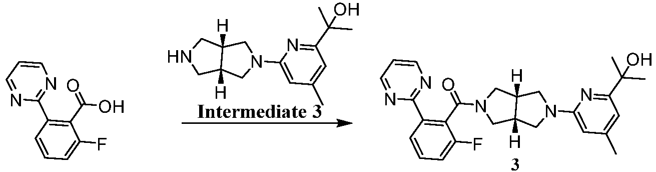

2-fluoro-6-pyrimidin-2-ylbenzoic acid (50 mg, 0.23 mmol) and intermediate 3 (65.88 mg, 0.25 mmol) were added to MeCN (5 mL). Tetramethylchlorourea hexafluorophosphate (128.33 mg, 0.46 mmol) and 1-methylimidazole (56.45 mg, 0.69 mmol) were added at 25°C. The reaction solution was stirred at 25°C for 2 hours, water (20 mL) was added, and the mixture was extracted with dichloromethane (30 mL*2). The combined extracts were dried over Na₂SO₄ and spun to dryness, and the crude product was purified by preparative HPLC to afford compound 3 (64 mg, 60.5% yield).
MS m/z (ESI): 462.2 [M+1]
¹H NMR (400 MHz, DMSO-d6) δ 8.93 (d, J = 4.9 Hz, 1H), 8.82 (d, J = 4.9 Hz, 1H), 8.05 (dd, J = 25.0, 7.8 Hz, 1H), 7.62 (q, J = 7.7 Hz, 1H), 7.54 - 7.38 (m, 2H), 6.85 (d, J = 9.8 Hz, 1H), 6.68 (d, J = 22.0 Hz, 1H), 3.80 (ddd, J = 37.8, 12.6, 7.8 Hz, 4H), 3.61 - 3.51 (m, 3H), 3.18 (dt, J = 10.8, 5.1 Hz, 3H), 2.36 (d, J = 3.4 Hz, 3H), 1.50 (d, J = 5.2 Hz, 6H).

### Example 4

### Preparation of compound 4: (2-fluoro-6-(pyrimidin-2-yl)phenyl)((3aR,6aS)-5-(6-(2-fluoropropan-2-yl)-4-methylpyridin-2-yl)hexahydropyrrolo[3,4-c]pyrrol-2(1H)-yl)methanone

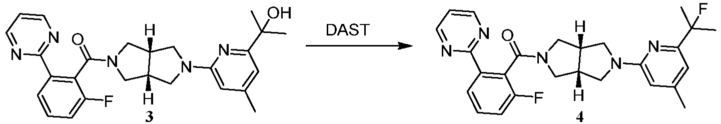

Compound 3 (45 mg, 0.1 mmol) and DAST (23.57 mg, 0.15 mmol) were added to DCM (5 mL) at 0°C. The reaction solution was stirred at 25°C for 0.5 hours. After the reaction was completed, water (20 ml) was added, and the mixture was extracted with dichloromethane (30 mL*2). The organic phases were combined, dried over Na₂SO₄ and spun to dryness, and the crude product was purified by preparative HPLC to afford compound 4 (31 mg, 68.6% yield).
MS: m/z (ESI): 464.2 [M+1].
¹H NMR (400 MHz, DMSO-d6) δ 8.92 (d, J = 4.9 Hz, 1H), 8.76 (d, J = 4.9 Hz, 1H), 8.03 (dd, J = 31.4, 7.8 Hz, 1H), 7.67 - 7.33 (m, 3H), 6.56 (d, J = 3.8 Hz, 1H), 6.20 (d, J = 5.4 Hz, 1H), 3.85 - 3.65 (m, 2H), 3.59 - 3.47 (m, 3H), 3.27 - 2.90 (m, 5H), 2.23 (s, 3H), 1.60 (dd, J = 22.0, 3.0 Hz, 6H).

### Example 5

### Preparation of compound 5: ((3aR,6aS)-5-(5-fluoro-4-(2-fluoropropan-2-yl)-6-methylpyrimidin-2-yl)hexahydropyrrolo[3,4-c]pyrrol-2(1H)-yl)(2-fluoro-6-(2H-1,2,3-triazol-2-yl)phenyl)methanone

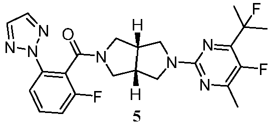

With reference to the synthesis method of Example 4, compound 5 was synthesized by using compound 2 as raw material instead of compound 3, to afford compound 5 (30 mg, 66% yield).
MS: m/z (ESI): 472.2 [M+1].
¹H NMR (400 MHz, DMSO-d6) δ 8.16 (s, 1H), 7.96 (s, 1H), 7.80 (dd, J = 14.7, 8.2 Hz, 1H), 7.66 (td, J = 8.3, 6.0 Hz, 1H), 7.44 (q, J = 9.2 Hz, 1H), 3.76 - 3.64 (m, 2H), 3.53 (ddd, J = 23.2, 11.1, 7.2 Hz, 5H), 3.17 - 2.94 (m, 3H), 2.36 - 2.23 (m, 3H), 1.67 (dd, J = 21.9, 4.0 Hz, 6H).

### Example 6

### Preparation of compound 6: (2-fluoro-6-(2H-1,2,3-triazol-2-yl)phenyl)((3R,6S)-5-(6-(2-hydroxypropan-2-yl)-4-methylpyridin- 2-yl)hexahydropyrrolo[3,4-c]pyrrol-2(1H)-yl)methanone

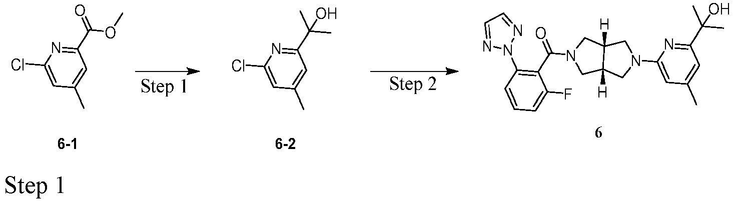

Compound 6-1 (100.00 mg, 539.08 µmol) was dissolved in tetrahydrofuran (5.0 mL), methylmagnesium bromide (3 M, 538.77 µL) was added to the reaction liquid, and reaction liquid was reacted under stirring at 0°C for 0.5 h. Saturated ammonium chloride (5 ml) was added to the reaction liquid, the mixture was extracted with dichloromethane (3* 10 mL), and the organic phases were combined, dried, and concentrated to obtain a crude product. The crude product was purified by column chromatography (petroleum ether/ethyl acetate system) to obtain product 2-(6-chloro-4-methylpyridin-2-yl)propan-2-ol (compound 6-2) (90 mg, 90.0%).
MS m/z (ESI): 186.1 [M+1]

### Step 2

Compound 6-2 (65.95 mg, 0.36 mmol), compound 3c (100 mg, 0.33 mmol), 4,5-bisdiphenylphosphino-9,9-dimethylxanthene (34.22 mg, 59.20 µmol) and palladium acetate (6.63 mg, 29.61 µmol) were dissolved in 1,4-dioxane (2 mL), potassium tert-butoxide (33.16 mg, 296.07 µmol) was added to the reaction liquid, and then the reaction liquid was reacted under stirring under microwave at 100°C for 1 h. Saturated aqueous sodium chloride solution (10 ml) was added to reaction liquid, the mixture was extracted with dichloromethane (3*10 mL), and the organic phases were combined, dried, and concentrated to obtain a crude product. The crude product was subjected to preparative purification to obtain (2-fluoro-6-(2H-1,2,3-triazol-2-yl)phenyl)((*3R,6S*)-5-(6-(2-hydroxypropan-2-yl)-4-methylpyridin-2-yl)hexahydropyrrolo[3,4-c]pyrrol-2(1H)-yl)methanone (compound 6) (8 mg, 6.00% yield).
MS m/z (ESI): 451.1 [M+1].
¹H NMR (400 MHz, DMSO) δ 7.95 (s, 1H), 7.80 (dd, J = 18.7, 8.2 Hz, 1H), 7.73 - 7.55 (m, 1H), 7.40 (m, 1H), 7.02 (dd, J = 58.3, 50.0 Hz, 2H), 6.66 (d, J = 6.9 Hz, 1H), 6.11 (s, 1H), 3.87 - 3.48 (m, 7H), 3.27 - 2.90 (m, 3H), 2.21 (d, J = 4.9 Hz, 3H), 1.38 (d, J = 6.3 Hz, 6H).

### Example 7

### Preparation of compound 7: ((3aR,6aS)-5-(5-fluoro-4-(2-hydroxyprop-2-yl)pyrimidin-2-yl)hexahydropyrrolo[3,4-c]pyrrol-2(1H)-yl)(2-fluoro-6-(2H-1,2,3-triazol-2-yl)phenyl)methanone

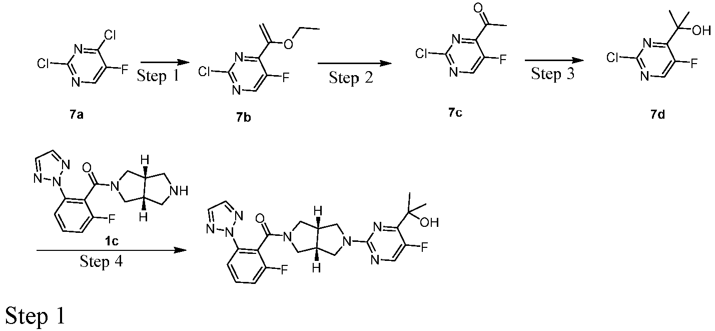

Tributyl(1-ethoxyethenyl)stannane (11.90 g, 32.95 mmol) and compound 7a (5 g, 29.95 mmol) were dissolved in DMF (5 mL), bis(triphenylphosphine)palladium(II) chloride (1.05 g, 1.50 mmol) was added, and the reaction liquid was reacted under stirring at 100°C for 2 h. After the reaction was completed, saturated aqueous potassium fluoride solution (10 ml) was added, and the mixture was stirred for 1 h and filtered. The filter cake was washed with dichloromethane (10 mL*3), the organic phases were combined, dried, and concentrated to afford a crude product, and the crude product was purified by column chromatography (petroleum ether /ethyl acetate system) to afford compound 7b (5.6 g, 92.3% yield). MS m/z (ESI): 203.0 [M+1]

### Step 2

Compound 7b (5.6 g, 27.64 mmol) was dissolved in tetrahydrofuran (2 mL), hydrochloric acid (2 mL, 27.64 mmol) was added to the reaction liquid, and then the reaction liquid was reacted under stirring at 50°C for 2 h. Saturated sodium chloride (10 mL) was added to the reaction liquid, the mixture was extracted with dichloromethane (10 mL*3), and the organic phases were combined, dried, and concentrated to afford a crude product. The crude product was purified by column chromatography (petroleum ether/ethyl acetate system) to afford compound 7c (4.4 g, 91.2% yield).
MS m/z (ESI): 175.0 [M+1]

### Step 3

Compound 7c (4.4 g, 25.21 mmol) was dissolved in tetrahydrofuran (5 mL), methylmagnesium bromide (3 M, 9.24 mL) was added to the reaction liquid at - 78°C, and then the reaction liquid was reacted under stirring at -78°C for 15 min. Saturated ammonium chloride (10 mL) was added to the reaction liquid, the mixture was extracted with dichloromethane (10 mL*3), and the organic phases were combined, dried, and concentrated to afford a crude product. The crude product was purified by column chromatography (petroleum ether /ethyl acetate system) to afford 7d (2.2 g, 45.8% yield).
MS m/z (ESI): 191.0 [M+1]

### Step 4

With reference to the synthesis method of compound 6, compound 7 was synthesized by using compound 1c and compound 7d as raw materials, to afford compound 7 (38 mg, 42.6% yield).
MS: m/z (ESI): 456.2 [M+1]
¹H NMR (400 MHz, DMSO) δ 8.21 (t, J = 23.7 Hz, 1H), 7.99 (d, J = 73.9 Hz, 2H), 7.73 (dd, J = 17.8, 8.2 Hz, 1H), 7.59 (dd, J = 14.6, 8.1 Hz, 1H), 7.37 (dd, J = 19.5, 8.8 Hz, 1H), 5.13 (d, J = 9.5 Hz, 1H), 3.91 - 3.34 (m, 7H), 3.14 - 2.87 (m, 3H), 1.39 (d, J = 4.2 Hz, 6H).

### Example 8

### Preparation of compound 8: (2-fluoro-6-(pyrimidin-2-yl)phenyl)((3R,6S)-5-(6-(2-hydroxypropan-2-yl)-5-methylpyridin-2-yl)hexahydropyrrolo[3, 4-c]pyrrol-2(1H)-yl)methanone

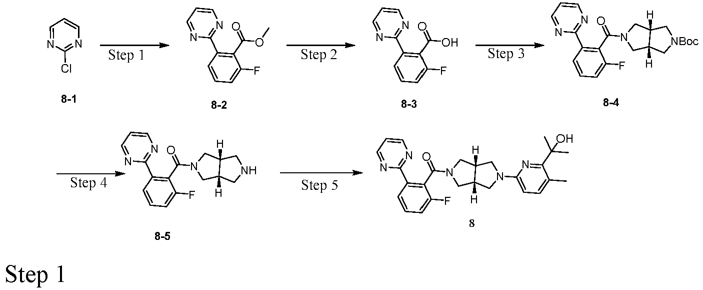

Compound 8-1 (0.2 g, 1.75 mmol), methyl 2-fluoro-6-(4,4,5,5-tetramethyl-1,3,2-dioxaborolan-2-yl)benzoate (586.94 mg, 2.10 mmol) and potassium phosphate (1.11 g, 5.24 mmol) were dissolved in 1, 4-dioxane (4.0 mL) and H₂O (1.0 mL), [1,1'-bis(diphenylphosphino)ferrocene]palladium dichloride dichloromethane complex (142.49 mg, 174.62 µmol) was added to the reaction liquid, and then the reaction liquid was reacted under stirring at 100°C for 16 h. Saturated aqueous sodium chloride solution (10 ml) was added to the reaction liquid, the mixture was extracted with dichloromethane (3*20 mL), and the organic phases were combined, dried, and concentrated to obtain a crude product. The crude product was purified by column chromatography (petroleum ether/ethyl acetate system) to obtain methyl 2-fluoro-6-(pyrimidin-2-yl)benzoate (compound 8-2) (0.2 g, 49.1%).
MS m/z (ESI): 233.1 [M+1]

### Step 2

Compound 8-2 (0.8 g, 3.45 mmol) was dissolved in water (2 mL) and methanol (2 mL), sodium hydroxide (413.39 mg, 10.33 mmol) was added to the reaction liquid, and then the reaction liquid was reacted under stirring at 70°C for 16 h. The reaction liquid was adjusted pH to acidic with hydrochloric acid solution (2 mol/L, 10 ml), and extracted with ethyl acetate (3*20 mL), and the organic phases were combined, dried, and concentrated to obtain crude product 2-fluoro-6-(pyrimidin-2-yl)benzoic acid (compound 8-3) (260 mg, 34.6%). The crude product was used directly in the next step without purification.
MS m/z (ESI): 219.1 [M+1]

### Step 3

Compound 8-3 (0.2 g, 917.43 µmol) and N,N-dimethylformamide (0.1 ml) were dissolved in dichloromethane (2 mL), and then oxalyl chloride (232.70 mg, 1.83 mmol) was added to the reaction liquid. The reaction liquid was reacted under stirring at room temperature for 0.5 h, and the reaction liquid was concentrated to obtain a crude product. The crude product, tert-butyl (3R,6S)-2,3,3a,4,6,6a-hexahydro-1H-pyrrolo[3,4-c]pyrrole -5-carboxylate (192.88 mg, 909.81 µmol) was dissolved in dichloromethane (2 mL), N-ethyl-N-isopropylpropan-2-amine (352.29 mg, 2.73 mmol) was added to the reaction liquid, and then the reaction liquid was reacted under stirring at room temperature for 1 h. Saturated aqueous sodium chloride solution (10 ml) was added to the reaction liquid, the mixture was extracted with dichloromethane (3*10 mL), and the organic phases were combined, dried, and concentrated to obtain a crude product. The crude product was purified by column chromatography (petroleum ether/ethyl acetate system) to obtain tert-butyl (*3R,6S*)-5-(2-fluoro-6-(pyrimidin-2-yl)benzoyl)hexahydropyrrolo[3,4-c]pyrrole-2(1H)-carboxylate (compound 8-4) (0.2 g, 53.4%).
MS m/z (ESI): 413.2 [M+1]

### Step 4

Compound 8-4 (200.00 mg, 485.43 µmol) was dissolved in hydrochloric acid/1,4-dioxane (4 M, 2 mL), and then the reaction liquid was reacted under stirring at room temperature for 1 h. The reaction liquid was filtered, and the filter cake was collected and dried to obtain (2-fluoro-6-(pyrimidin-2-yl)phenyl)((*3R,6S*)-hexahydropyrrolo[3,4-c]pyrrol-2(1H)-yl)methanone (compound 8-5) (120 mg, 79.2%).
MS m/z (ESI): 313.2 [M+1]

### Step 5

With reference to the synthesis method of compound 6, compound 8 was synthesized by using compound 8-5 and compound 2-(6-chloro-3-methylpyridin-2-yl)-propan-2-ol as raw materials, to afford (2-fluoro-6-(pyrimidin-2-yl)phenyl)((*3R,6S*)-5-(6-(2-hydroxypropan-2-yl)-5-methylpyridin-2-yl)hexahydropyrrolo[3, 4-c]pyrrol-2(1H)-yl)methanone (compound 8) (1.8 mg, 2.6%).
MS: m/z (ESI): 462.2 [M+1]

### Example 9

### Preparation of compound 9: ((3aR,6aS)-5-(5-fluoro-4-(2-hydroxyprop-2-yl)pyrimidin-2-yl)hexahydropyrrolo[3,4-c]pyrrol-2(1H)-yl)(2-fluoro-6-(pyrimidin-2-yl)phenyl)methanone

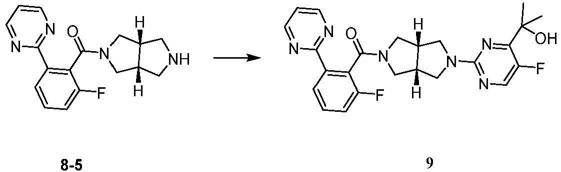

With reference to the synthesis method of compound 6, compound 9 was synthesized by using compounds 8-5 and 7d as raw materials, to afford compound 9 (22 mg, 28.6% yield).
MS: m/z (ESI): 467.2 [M+1]
¹H NMR (400 MHz, DMSO) δ 8.86 (dd, J = 48.0, 4.9 Hz, 2H), 8.32 (s, 1H), 8.02 (dd, J = 35.7, 7.8 Hz, 1H), 7.60 (dd, J = 14.0, 7.9 Hz, 1H), 7.44 (ddd, J = 26.7, 12.9, 4.9 Hz, 2H), 5.33 - 5.10 (m, 1H), 3.89 - 3.46 (m, 7H), 3.23 - 2.95 (m, 3H), 1.46 (s, 6H).

### Example 10

### Preparation of compound 10: (4-fluoro-2-(2H-1,2,3-triazol-2-yl)phenyl)((cis)-5-(5-fluoro-4-(2-hydroxypropan-2-yl)-6-methylpyrimidin-2-yl)hexahydropyrrolo [3,4-c]pyrrol-2(1H)-yl)methanone

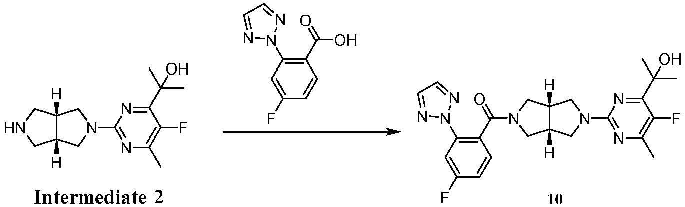

Intermediate 2 (80 mg, 0.285 mmol) was dissolved in acetonitrile (2 mL), 4-fluoro-2-(triazol-2-yl)benzoic acid (59 mg, 0.285 mmol), N-methylmorpholine (36 mg, 0.356 mmol) and N,N,N',N'-tetramethylchloroformamidinium hexafluorophosphate (120 mg, 0.428 mmol) were added, and the reaction liquid was reacted at room temperature for 2 hours. After the reaction was completed, the reaction liquid was poured into water (50 mL) and the mixture was extracted with ethyl acetate (30 mL*2). The organic phases were combined, washed with water (30 mL) and saturated sodium chloride solution (30 mL) in sequence, dried over anhydrous sodium sulphate and filtered, the filtrate was concentrated under reduced pressure, and the residue was purified by reverse-phase preparative HPLC chromatography (acetonitrile/water system) to afford compound 10 (16.3 mg, 12.2% yield).
MS m/z (ESI): 470.2 [M+1]
¹H NMR (400 MHz, DMSO-*d*₆) δ 8.03 (s, 2H), 7.74 (dd, *J =* 9.6, 2.0 Hz, 1H), 7.60 - 7.56 (m, 1H), 7.43 - 7.38 (m, 1H), 5.17 (s, 1H), 3.75 - 3.65 (m, 2H), 3.60 - 3.56 (m, 1H), 3.48 - 3.44 (m, 3H), 3.34 - 3.29 (m, 1H), 3.02 - 2.93 (m, 3H), 2.31 (s, 3H), 1.46 (s, 6H).

### Example 11

### Preparation of compound 11: (3-fluoro-2-(2H-1,2,3-triazol-2-yl)phenyl)((cis)-5-(5-fluoro-4-(2-hydroxypropan-2-yl)-6-methylpyrimidin-2-yl)hexahydropyrrolo [3,4-c]pyrrol-2(1H)-yl)methanone

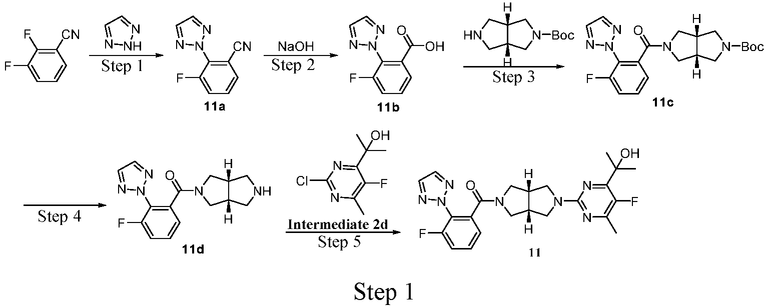

2,3-Difluorobenzonitrile (12.0 g, 86.3 mmol) was dissolved in N,N-dimethylformamide (100 mL), 1,2,3-triazole (5.96 g, 86.3 mmol) and caesium carbonate (28.1 g, 86.3 mmol) were added, and the reaction liquid was heated to 120°C and reacted for 2 hours. After the reaction was completed, the reaction liquid was cooled to room temperature and poured into water (200 mL), and the mixture was extracted with methyl tert-butyl ether (100 mL*2). The organic phases were combined, washed with water (100 mL) and saturated sodium chloride solution (100 mL) in sequence, dried over anhydrous sodium sulphate and filtered, the filtrate was concentrated under reduced pressure, and the residue was purified by silica gel column chromatography (petroleum ether/ethyl acetate system) to afford compound 11a (6.5 g, 40.0% yield).
MS m/z (ESI): 189.0 [M+1]
¹H NMR (400 MHz, CDCl₃) δ 8.00 (s, 2H), 7.67 - 7.65 (m, 1H), 7.62 - 7.55 (m, 2H).

### Step 2

Compound 11a (1.5 g, 7.97 mmol) was dissolved in 1,4-dioxane (10 mL) and water (30 mL), sodium hydroxide (3.19 g, 79.8 mmol) was added, and the mixture was heated to 110°C and reacted for 4 hours. The reaction liquid was cooled to 0°C, adjusted to pH about 1 with dilute hydrochloric acid, and extracted with ethyl acetate (100 mL*2). The organic phases were combined, washed with water (100 mL) and saturated sodium chloride solution (100 mL) in sequence, dried over anhydrous sodium sulphate and filtered, and the filtrate was concentrated under reduced pressure, to afford compound 11b (1.5 g, 90.8% yield).
MS m/z (ESI): 208.0 [M+1]
¹H NMR (400 MHz, CDCl₃) δ 7.89 (s, 2H), 7.87 (d, *J =* 7.6 Hz, 1H), 7.63 - 7.58 (m, 1H), 7.52 - 7.49 (m, 1H).

### Step 3

(Cis)-2-Boc-hexahydropyrrolo[3,4-c]pyrrole (120 mg, 0.565 mmol) was dissolved in anhydrous acetonitrile (2 mL), compound 11b (117 mg, 0.565 mmol), N,N,N',N'-tetramethylchloroformamidinium hexafluorophosphate (238 mg, 0.848 mmol) and N-methyl morpholine (86 mg, 0.850 mmol) were added, and the mixture was reacted at room temperature for 2 hours. The reaction liquid was poured into water (30 mL) and the mixture was extracted with ethyl acetate(30 mL*2). The organic phases were combined, washed with water (80 mL) and saturated sodium chloride solution (80 mL) in sequence, dried over anhydrous sodium sulphate and filtered, the filtrate was concentrated under reduced pressure, and the residue was purified by silica gel column chromatography (petroleum ether/ethyl acetate system) to afford compound 11c (210 mg, 92.6% yield).
MS m/z (ESI): 402.2 [M+1]
¹H NMR (400 MHz, DMSO-*d*₆) δ 8.07 (s, 2H), 7.71 - 7.66 (m, 1H), 7.63 - 7.58 (m, 1H), 7.42 (d, *J =* 7.6 Hz, 1H), 3.49 - 3.42 (m, 3H), 3.40 - 3.36 (m, 1H), 3.23 - 3.19 (m, 1H), 3.16 - 3.01 (m, 3H), 2.87 - 2.78 (m, 2H), 1.41 (s, 9H).

### Step 4

Compound 11c (80 mg, 0.199 mmol) was dissolved in anhydrous dichloromethane (2 mL), a solution of hydrochloric acid in dioxane (4 M, 2 mL) was added, and the mixture was reacted at room temperature for 2 hours. The reaction liquid was concentrated under reduced pressure, to afford compound 11d (60 mg, 99.9% yield).
MS m/z (ESI): 302.1 [M+1]

### Step 5

Compound 11d (60 mg, 0.199 mmol) was dissolved in anhydrous N,N-dimethylformamide (2 mL), intermediate 2d (45 mg, 0.220 mmol) and caesium carbonate (130 mg, 0.399 mmol) were added, and the mixture was heated to 110°C and reacted for 2 hours. The reaction liquid was cooled to room temperature and poured into water (30 mL), and the mixture was extracted with ethyl acetate (30 mL*2). The organic phases were combined, washed with water (30 mL) and saturated sodium chloride solution (30 mL) in sequence, dried over anhydrous sodium sulphate and filtered, the filtrate was concentrated under reduced pressure, and the residue was purified by reverse-phase preparative HPLC chromatography (acetonitrile/water system) to afford compound 11 (31.3 mg, 33.5% yield).
MS m/z (ESI): 470.2 [M+1]
¹H NMR (400 MHz, DMSO-*d*₆) δ 8.00 (s, 2H), 7.70 - 7.65 (m, 1H), 7.62 - 7.57 (m, 1H), 7.42 (d, *J =* 7.6 Hz, 1H), 5.16 (s, 1H), 3.73 - 7.69 (m, 1H), 3.64 - 3.59 (m, 1H), 3.57 - 3.49 (m, 2H), 3.43 - 3.34 (m, 2H), 3.29 - 3.25 (m, 1H), 3.13 - 3.09 (m, 1H), 2.99 - 2.93 (m, 2H), 2.32 (d, *J =* 2.8 Hz, 3H), 1.46 (s, 6H).

### Example 12

### Preparation of compound 12: ((3aR,6aS)-5-(5-fluoro-4-(2-hydroxypropan-2-yl)-6-methylpyrimidin-2-yl)hexahydropyrrolo[3,4-c]pyrrol-2(1H)-yl)(2-fluoro-6-(pyridin-2-yl)phenyl)methanone

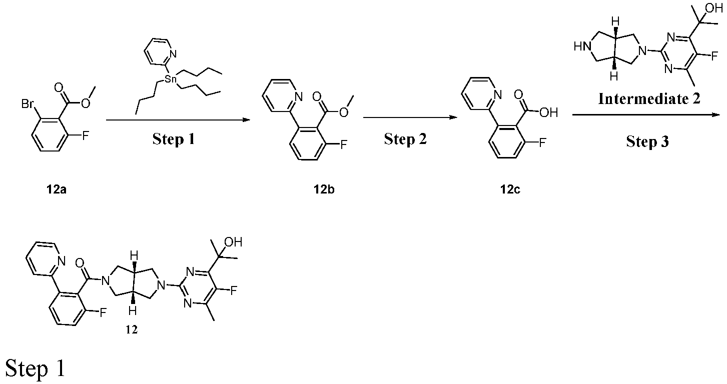

Under N₂ protection, to a solution of compound 12a (4 g, 17.16 mmol) and 2-(tributylstannyl)pyridine (6.97 g, 18.93 mmol) in DMF (60 mL) were added CuI (653.81 mg, 3.43 mmol), Pd(PPh₃)₄ (1.98 g, 1.71 mmol) and CsF (5.21 g, 34.30 mmol). The mixture was reacted at 120°C for 5 hours. The mixture was cooled to room temperature, water (150 mL) was added, and the mixture was extracted with ethyl acetate (100 mL*2). The organic phases were combined, dried over anhydrous sodium sulphate, filtered and concentrated. The obtained crude product was purified to afford compound 12b (2.8 g, yield 70.5%).
MS m/z (ESI): 232.1 [M+1]

### Step 2

To a mixed solution of compound 12b (800 mg, 3.46 mmol) in water (5.0 mL) and methanol (5.0 mL) was added sodium hydroxide (688.98 mg, 17.22 mmol). The mixture was reacted at 25°C for 3 hours. 2 N HCl was added until pH = 2, and the mixture was extracted with ethyl acetate (20 mL*2). The organic phase was dried over anhydrous sodium sulphate, filtered and spun to dryness, to afford compound 12c (650 mg, 86.5% yield).
MS m/z (ESI): 218.0 [M+1]

### Step 3

To a solution of 12c (100 mg, 0.46 mmol) and intermediate 2 (129 mg, 0.46 mmol) in acetonitrile (3.0 mL) were added tetramethylchlorourea hexafluorophosphate (257 mg, 0.92 mmol) and N-methyl imidazole (188 mg, 2.29 mmol). The mixture was stirred at 25°C for 30 minutes. To the reaction liquid was added water (20 mL), and the mixture was extracted with ethyl acetate (20 mL*2). The organic phases were combined, dried over anhydrous sodium sulphate, filtered and concentrated. The obtained crude product was purified by preparative HPLC, to afford compound 12 (40 mg, 18.1% yield).
MS m/z (ESI): 480.2 [M+1]
¹H NMR (400 MHz, DMSO-d6) δ 8.64 - 8.44 (m, 1H), 7.87 (dq, J = 21.9, 7.1, 6.6 Hz, 1H), 7.76 - 7.48 (m, 3H), 7.43 - 7.19 (m, 2H), 5.16 (s, 1H), 3.67 (dddd, J = 38.5, 25.2, 11.5, 7.2 Hz, 5H), 3.23 - 2.95 (m, 5H), 2.32 (dd, J = 11.6, 2.8 Hz, 3H), 1.46 (d, J = 11.0 Hz, 6H).

### Example 13

### Preparation of compound 13: (5-fluoro-2-(2H-1,2,3-triazol-2-yl)phenyl)((cis)-5-(5-fluoro-4-(2-hydroxypropan-2-yl)-6-methylpyrimidin-2-yl)hexahydropyrrolo [3,4-c]pyrrol-2(1H)-yl)methanone

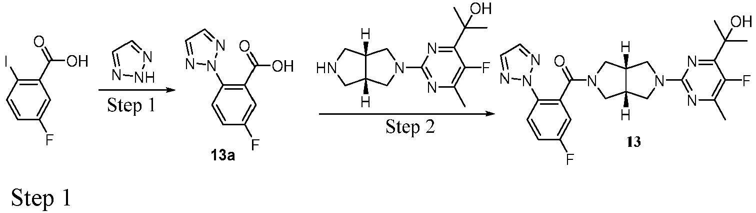

5-fluoro-2-iodobenzoic acid (300 mg, 1.13 mmol) was dissolved in anhydrous N,N-dimethylformamide (4 mL), 1,2,3-triazole (117 mg, 1.69 mmol), cuprous iodide (258 mg, 1.35 mmol), trans-N,N'-dimethyl-1,2-cyclohexanediamine (193 mg, 0.97 mmol) and caesium carbonate (735 mg, 2.26 mmol) were added, and the mixture was reacted under microwave at 120°C for 15 minutes. The reaction liquid was cooled to room temperature and poured into water (50 mL), the mixture was adjusted to pH 5 with dilute hydrochloric acid, and extracted with ethyl acetate (50 mL*2). The organic phases were combined, washed with water (50 mL) and saturated sodium chloride solution (50 mL) in sequence, dried over anhydrous sodium sulphate and filtered, the filtrate was concentrated under reduced pressure, and the residue was purified by reverse-phase C18 column chromatography (acetonitrile/water system) to afford compound 13a (150 mg, 64.2% yield).

MS m/z (ESI): 208.0 [M+1].

### Step 2

With reference to the preparation method of step 3 of compound 12, compound 13 was prepared with compound 13a instead of compound 12c, to afford compound 13 (60 mg, 30% yield).

MS m/z (ESI): 470.2 [M+1].

¹H NMR (400 MHz, DMSO-*d*₆) δ 7.98 (s, 2H), 7.93 - 7.89 (m, 1H), 7.49 - 7.46 (m, 1H), 7.37 - 7.35 (m, 1H), 5.16 (s, 1H), 3.81 - 3.57 (m, 3H), 3.42 - 3.37 (m, 4H), 3.04 - 2.94 (m, 3H), 2.30 (s, 3H), 1.46 (s, 6H).

### Example 14

### Preparation of compound 14: (5-fluoro-2-(pyrimidin-2-yl)phenyl)((cis)-5-(5-fluoro-4-(2-hydroxypropan-2-yl)-6-methylpyrimidin-2-yl)hexahydropyrrolo[3,4-c]pyrrol-2(1H)-yl)methanone

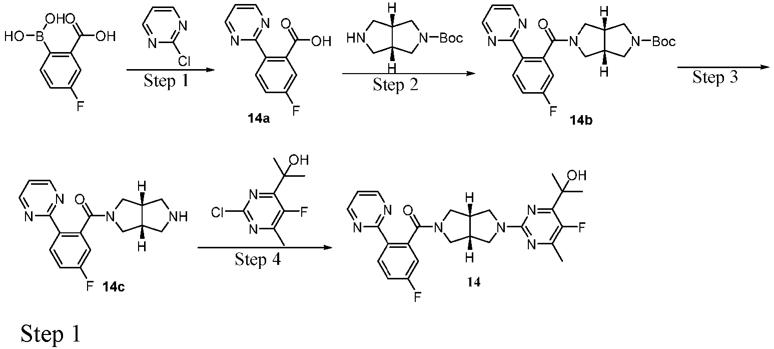

2-Chloropyrimidine (280 mg, 2.44 mmol) was dissolved in anhydrous N,N-dimethylformamide (4 mL) and water (1 mL), 2-borono-5-fluorobenzoic acid (300 mg, 1.63 mmol), tetrakis(triphenylphosphine)palladium (86 mg, 0.074 mmol) and caesium carbonate (451 mg, 1.38 mmol) were added, and the mixture was heated to 100°C and reacted for 2 hours under nitrogen protection. The reaction liquid was cooled to room temperature, water (15 mL) was added, and the mixture was filtered. The filtrate was concentrated under reduced pressure, ethanol (10 mL) was added, and the mixture was filtered to remove inorganic salt. The filtrate was concentrated under reduced pressure, to afford crude product compound 14a (260 mg, 73.1% yield), which was directly used in the next reaction.

MS m/z (ESI): 219.1 [M+1].

### Step 2

The preparation method of compound 14b referred to the preparation method of compound 11c.

MS m/z (ESI): 413.2 [M+1].

### Step 3

The preparation method of compound 14c referred to the preparation method of compound 11d.

MS m/z (ESI): 313.1 [M+1].

### Step 4

The preparation method of compound 14 referred to the preparation method of compound 11.

MS m/z (ESI): 481.2 [M+1].

¹H NMR (400 MHz, DMSO-*d*₆) δ 8.81 (d, *J =* 4.8 Hz, 2H), 8.19 (dd, *J =* 8.8, 5.6 Hz, 1H), 7.42 - 7.37 (m, 2H), 7.31 - 7.29 (m, 1H), 5.16 (s, 1H), 3.78 - 3.67 (m, 2H), 3.62 - 3.46 (m, 5H), 3.09 - 2.95 (m, 3H), 2.31 (d, *J* = 2.4 Hz, 3H), 1.46 (s, 6H).

### Example 15

### Preparation of compound 15: ((3aR,6aS)-5-(5-fluoro-4-(2-hydroxypropan-2-yl)-6-methylpyrimidin-2-yl)hexahydropyrrolo[3,4-c]pyrrol-2(1H)-yl)(2-fluoro-6-(pyrimidin-2-yl)phenyl)methanone

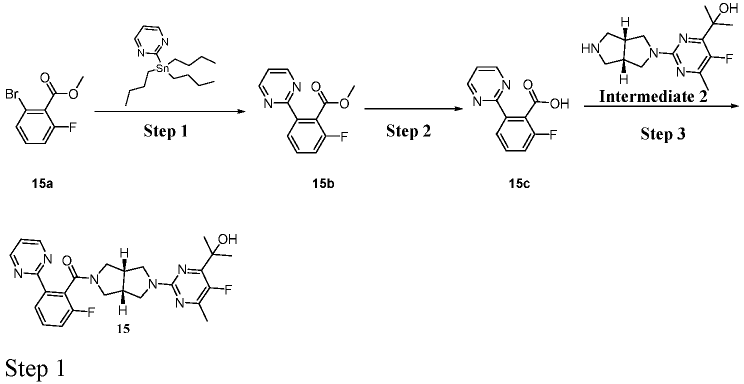

Under N₂ protection, to a solution of compound 15a (4 g, 17.16 mmol) and 2-(tributylstannyl)pyrimidine (6.97 g, 18.88 mmol) in DMF(60 mL) were added CuI (653.81 mg, 3.43 mmol), Pd(PPh₃)₄ (1.98 g, 1.71 mmol) and CsF (5.21 g, 34.30 mmol). The mixture was reacted at 120°C for 5 hours. The mixture was cooled to room temperature, water (150 mL) was added, and the mixture was extracted with ethyl acetate (100 mL*2). The organic phases were combined, dried over anhydrous sodium sulphate, filtered and concentrated. The obtained crude product was purified to afford compound 15b (2.8 g, yield 70.3%).

MS m/z (ESI): 233.1 [M+1].

### Step 2

To a mixed solution of compound 15b (800 mg, 3.45 mmol) in water (5.0 mL) and methanol (5.0 mL) was added sodium hydroxide (688.98 mg, 17.22 mmol). The mixture was reacted at 25°C for 3 hours. 2 N HCl was added until pH = 2, and the mixture was extracted with ethyl acetate (20 mL*2). The organic phase was dried over anhydrous sodium sulphate, filtered and spun to dryness, to afford compound 15c (650 mg, 86.5% yield).

MS m/z (ESI): 219.0 [M+1].

### Step 3

To a solution of 106c (100 mg, 0.46 mmol) and intermediate 2 (129 mg, 0.46 mmol) in acetonitrile (3.0 mL) were added tetramethylchlorourea hexafluorophosphate (257 mg, 0.92 mmol) and N-methyl imidazole (188 mg, 2.29 mmol). The mixture was stirred at 25°C for 30 minutes. To the reaction liquid was added water (20 mL), and the mixture was extracted with ethyl acetate (20 mL*2). The organic phases were combined, dried over anhydrous sodium sulphate, filtered and concentrated. The obtained crude product was purified by preparative HPLC, to afford compound 15 (40 mg, 18.2% yield).

MS m/z (ESI): 481.2 [M+1].

¹H NMR (400 MHz, DMSO-d6) δ 8.85 (dd, J = 42.9, 4.9 Hz, 2H), 8.01 (dd, J = 36.2, 7.8 Hz, 1H), 7.59 (td, J = 8.1, 5.8 Hz, 1H), 7.51 - 7.37 (m, 2H), 5.15 (s, 1H), 3.85 - 3.47 (m, 7H), 3.09 (dddd, J = 36.1, 22.9, 11.1, 7.0 Hz, 3H), 2.36 - 2.24 (m, 3H), 1.44 (d, J = 3.3 Hz, 6H).

### Biological test and evaluation

The present invention will be further described and explained below in conjunction with test examples, but these examples are not meant to limit the scope of the present invention.

### 1. Cell function experiment

Test example 1. Determination of the effect of the compounds of the present invention on calcium current in cells stably expressing OX1 and OX2 receptors
1. Experimental objective:
The inhibitory effect of the compounds on calcium current activity in CHO-K1/human OX1R and CHO-K1/human OX2R cells was detected.
2. Experimental instruments and reagents
2.1 Instruments:
384-well cell culture plate (Corning; 3764);
Plate reader FLIPR Tetra (Molecular Device).

2.2 Reagents:
DMEM, high glucose (Gibco: 12100);
Fetal bovine serum (Biosera: FB-1058/500);
P/S (Biosera: XC-A4122);
5X Matrigel (Corning: 354230);
HBSS (Sigma: H1387);
HEPES (Invitrogen: 15630080);
Fluo-8 AM (AAT Bioquest: 21080);
Probenecid (Sigma: P8761);
Pluronic F-127 (Sigma: P2443-250G);
1000X Fluo-8 AM (2 mM): Fluo-8 AM was dissolved in DMSO, the mixture was shaken for 1-2 min, sub-packaged and stored at -20°C;
Complete culture medium: DMEM + 10% FBS + 1X P/S;
Cell seeding medium: DMEM + 10% FBS + 1X PS;
Experimental buffer: 1X HBSS + 20 mM HEPES +1 mM Probenecid + 0.025% Pluronic F-127;
1X Matrigel: 5X Matrigel was diluted with DMEM;
Cell strain: CHO-K1/human OX1R and CHO-K1/human OX2R cell lines.

3. Experimental method:
1) CHO-K1/human OX1R and CHO-K1/human OX2R cell strains were cultured in complete culture medium at 37°C, 5% CO₂ to 70% to 90% confluence, respectively.
2) A 384-well cell culture plate was coated with 1X Matrigel at 5 uL/well at room temperature for 10 to 30 minutes.
3) The cells were digested and resuspended in cell seeding medium, and CHO-K1/human OX1R and CHO-K1/human OX2R cell strains were seeded into two 384-well cell culture plates at 8,000 cells/well/20 µL, respectively and cultured at 37°C, 5% CO₂ for 24 hours.
4) The cell culture plate was taken out of the CO₂ incubator and equilibrated at room temperature for 10 minutes.
5) 1000X Fluo-8 AM was taken and diluted to 1X Fluo-8 AM with experimental buffer 1 that has been equilibrated to room temperature, with a concentration of 2 µM.
6) The culture medium was removed from the cell culture plate, 20 µL of 1X Fluo-8 AM was added to each well, the mixture was centrifuged at 300 rpm for 60 seconds at room temperature, and incubated at room temperature in the dark for 1 hour.
7) EC80 of OX1 and OX2 receptor agonists was determined (OX-A for OX1R assays, OX-B for OX2R assays), in particular, the working solutions for diluting OX1 and OX2 receptor agonists were formulated, respectively; the diluted OX1 and OX2 receptor agonists were respectively added to the experimental wells of the corresponding 384-well cell culture plates of CHO-K1/human OX1R and CHO-K1/human OX2R cell strains and data was read and collected by using FLIPR Tetra, and the EC80 of OX-A and OX-B was obtained according to the following experimental data processing method, respectively.
8) Steps 1-7 were repeated, and positive compounds (suvorexant and seltorexant) and test compounds were formulated. The diluted compounds (11 concentration points) were added to the experimental wells of the corresponding two 384-well cell culture plates, and after 15 minutes, OX1 and OX2 receptor agonists were added to the well plates of the two cell strains according to the previously obtained EC80; and data was read and collected by using FLIPR Tetra. The IC50 of the positive compounds and test compounds was determined.

4. Experimental data processing method:
FLIPR Tetra was used to read and collect the fluorescence signal value (RFU). The maximum RFU value was used to calculate the percent inhibition (activation) data based on the readings of the low control (DMSO control) and high control (100 nM positive compound) experimental groups {% inhibition (activation) rate = (RFUsample - RFUlow control)/(RFUhigh control - RFUlow control) × 100}. Prism8 was used to fit the percentage inhibition (activation) rate and 11-point concentration data to a parametric nonlinear logic formula to calculate the IC₅₀ value of the compound.
5. Experimental results:

| Compound No. | OX1R IC50 (nM) | OX2R IC50 (nM) | Select fold |
|---|---|---|---|
| Seltorexant | 725 | 26.8 | 27 |
| 9 | 1062 | 8.7 | 122 |
| 10 | 1204 | 25.2 | 48 |
| 11 | 1324 | 27.0 | 49 |
| 12 | 263 | 7.26 | 36 |

The compound of the present invention showed a good inhibitory effect in the calcium current influence experiment in cells stably expressing the OX2 receptor, and the inhibitory effect of the compound on the OX2 receptor was significantly better than that of the OX1 receptor, and had good selectivity.

### 2. Pharmacokinetic evaluation test

### Rat pharmacokinetic evaluation test

### 1. Study objective:

SD rats were used as test animals to study the pharmacokinetic behaviour of the compounds of the present invention in rat (plasma) after oral administration at a dose of 5 mg/kg.

### 2. Experimental scheme:

### 2.1 Experimental drugs:

The example compound of the present invention, made in house.

### 2.2 Experimental animals:

3 SD rats/group, male, from Shanghai Jiesijie Experimental Animal Co., Ltd., animal production license number (SCXK (Shanghai) 2013-0006 N0.311620400001794).

### 2.3 Formulation prescription:

### Oral administration drug preparation: 20%HP-β-CD in Water

HP-β-CD powder (20 g) was weighed and dissolved in purified water (100 mL), and the mixture was homogeneously mixed by vortex and subjected to ultrasound treatment to afford a clear solution.

The example compound was weighed and added to a 20 mL glass bottle, the solution was added and the mixture was subjected to ultrasound treatment for 10 minutes to afford a colourless clear solution with a concentration of 0.5 mg/mL.

### 2.4 Administration:

3 male SD rats were administered p.o. respectively the solution after the rats were fasted overnight;
the p.o. dose was 5 mg/kg and the administration volume was 10 mL/kg.

### 2.5 Sample collection:

Blood collection: 0.2 mL of blood was collected from the jugular vein of rats before administration and at 0.25, 0.5, 1, 2, 4, 6, 8 and 24 h after administration, and placed in an EDTA-K₂ anticoagulation tube, and the plasma was separated by centrifugation at 6000 rpm at 4°C for 6 min and stored at -80°C; the rats were fed 4 h after administration.

Brain tissue collection: the experimental animals were euthanized by CO₂, and brain tissues were collected at 0.25 h, 0.5 h, 1 h, 2 h, 4 h, 6 h, 8 h, and 24 h after administration. The brain tissues were rinsed with pre-cooled PBS, wiped dry, weighed, and stored at -80°C.

### 2.6 Sample treatment:

1) Acetonitrile (160 µL) was added to plasma sample (40 µL) for precipitation, and the mixture was mixed and centrifuged at 3500 × g for 5 to 20 minutes.
2) The treated supernatant solution was taken and subjected to LC/MS/MS to analyse the concentrations of the test compounds. LC/MS/MS analysis instrument: AB Sciex API 4000 Qtrap.

### 2.7 Liquid phase analysis:

• Liquid phase conditions: Shimadzu LC-20AD pump
• Chromatographic column: Agilent ZORBAX XDB-C18 (50×2.1 mm, 3.5 µm) mobile phase: liquid A: 0.1% aqueous formic acid solution, liquid B: acetonitrile
• Flow rate: 0.4 mL/min
• Elution time: 0-4.0 min, the eluent is as follows:

| Time/min | Liquid A | Liquid B |
|---|---|---|
| 0.01 | 90% | 10% |
| 0.5 | 90% | 10% |
| 0.8 | 5% | 95% |
| 2.4 | 5% | 95% |
| 2.5 | 90% | 10% |
| 4.0 | Stop | |

### 3. Experimental results and analysis:

The main pharmacokinetic parameters were calculated using WinNonlin 6.1. The results of pharmacokinetic experiments in rats are as shown below:

| Number | Tmax (h) | AUC0-∞ (ng/mL*h) | Cmax (ng/mL) |
|---|---|---|---|
| | | Brain | Brain |
| Seltorexant | 0.25 | 79 | 130 |
| Example 9 | 0.6 | 130 | 124 |
| Example 11 | 0.25 | 821 | 394 |
| Example 12 | 0.5 | 335 | 420 |

### 4. Experimental conclusion:

Data showed that in the rat pharmacokinetic evaluation experiment, the example compounds of the present invention showed high exposure after oral administration, especially high exposure in the brain.

### 3. Evaluation of the effect of the compounds of the present invention on the sleep structure of rats by the telemetry electroencephalography and electromyography technique for sleep research

### 1. Experimental objective:

In this experiment, the telemetry electroencephalography and electromyography system of DSI was used to detect the effect of the compounds of the present invention on the sleep structure of SD rats.

### 2. Experimental instruments and reagents

### 2.1. Main instruments

DSI telemetry pressure measurement system: Implant model: TL11M2-F40-EET, Data Sciences International. HD-S02, Data Sciences International.
Data acquisition software: Ponemah Software 5.0, Data Sciences International.
Data analysis software: NeuroScore, Data Sciences International.
Precise electronic balance
Electronic balance
Ultrasonic device
Constant temperature magnetic stirrer
Vortex mixer

### 2.2. Main reagent

HP-β-CD

### 3. Experimental procedure

### 3.1. Purchase and adaptation of experimental animals

SD rats weighing 190-210 g upon arrival (5-6W) were used. Upon arrival, the rats were kept in animal housing rooms with strictly-controlled environmental conditions for an adaptation period of 7-9 days. During the adaptation period, the experimental animals were exposed to an environment with 12-h alternating light-dark cycle to adapt and adjust rhythm times, and the health status of the animals was monitored daily.

### 3.2. Grouping

All animals with the required body weight arrived at the animal facility and were adaptively raised, and electrodes were surgically implanted. The number of animals should be at least 6 per group, which met the requirements of statistical testing and pharmacodynamic guidelines.

### 3.3. Experimental process

1) Surgical implantation of electrodes: The experimental animals were exposed to an environment with 12-h alternating light-dark cycle and allowed to adapt for 7-9 days (lights off: 07:00; lights on: 19:00). On the day of the experiment, the animals were anaesthetised with Zoletil (i.p., 20 mg/kg) in combination with xylazine (i.p., 8 mg/kg). After anaesthesia, the head was fixed with a stereotaxic apparatus, and the surgical area of the head was prepared. The skull was drilled, and electrodes were implanted. Two electromyographic electrodes were inserted into the neck muscles in parallel at the same time, and the two ends were fixed with sutures to prevent the ends thereof from contacting each other. Then, the implant was placed under the skin of the back, and the surgical wound was sutured and disinfected.
2) Postoperative care: After surgery, the rats were carefully placed in a clean recovery cage in a lateral position to ensure that the airway was unobstructed. The experimental animals were placed in an environment with automatic 12-h alternating light-dark cycle (lights off: 07:00, lights on: 19:00) at constant temperature of 20°C-26°C and relative humidity of 40%-70%. After surgery, the animals were cared for 3 days. The surgical incision was administered locally cefradine powder, administered subcutaneously gentamicin at 4-8 mg/kg, and injected subcutaneously meloxicam at 0.1 ml/rat for 3 consecutive days. The experiment was carried out after 7-10 days of recovery, and the animals were randomly grouped according to body weight of the animals.
3) Administration regimen and indicator monitoring: Basic electroencephalography and electromyography recordings were performed 7-10 days after surgery. After the basic electroencephalography and electromyography recordings were completed, the administration began and continued for seven consecutive days. On day 1 and day 7 of administration, electroencephalography and electromyography recordings were performed 1 h before administration, i.e. at 11:00, the administration was performed at 12:00, and electroencephalography and electromyography recordings were performed 24 hours after administration.
4) Experimental indicators: Experimental indicators include time changes in NREM and REM latency between different groups after administration, and the time changes of Wake, NREM and REM within 24 hours after administration.

### 4. Data acquisition and analysis

The original data were collected by DSI system Ponemah software and analysed by NeuroScore software and GraphPad Prism software. The experimental data were expressed as mean ± standard error (Mean ± SEM), and statistical analysis was performed using ANOVA. P < 0.05 indicated significant differences.

### 5. Experimental results

The distribution of awake/sleep states of animals in the vehicle control group was consistent with the circadian rhythm. No significant effect of the vehicle on the sleep structure of animals was found, and the distribution of different awake/sleep stages was consistent with the literature reports. The model was successful and stable.

Each animal in the vehicle group and the administration group was analysed for different sleep stages from 1 h before administration to after administration. The mean value of each group was taken in units of 1 h, and the changes in the time distribution of different sleep stages of each group were analysed.

In the light-off stage, the total length of time that animals spend in different sleep stages 1 h, 3 h, 5 h, and 7 h after administration was counted, and pharmacodynamic effects in different administration groups were analysed.

In the light-on stage, the total length of time that animals spend in different sleep stages 1 h, 3 h, 5 h, and 7 h after light-on was counted, and pharmacodynamic effects in different administration groups were analysed.

### 6. Experimental conclusion

The example compounds of the present invention at a lower dose can significantly reduce the NREM/REM latency and the total times of wakefulness; significantly increase the total times of NREM, have no effect on the total REM time, and have a low risk of drowsiness. It can be seen that the compounds of the present invention have a good sleep-promoting effect on rats, and a low risk of drowsiness.

### 4. CYP enzyme single point inhibition test

### 1. Experimental objective

Human liver microsome incubation system was used, and the single point method was used to quickly predict the inhibition of compounds on CYP450 enzyme subtypes.

### 2. Experimental steps

### 2.1 Solution formulation

NADPH (reduced nicotinamide adenine dinucleotide phosphate) was weighed and added to 100 mM phosphate buffer, with the final concentration being 2.5 mM. 20 mg/mL microsomes (50 µL) was added to 100 mM phosphate buffer (4 mL), and the mixture was mixed until uniform to obtain 0.25 mg/mL microsomes.

Formulation of the reaction liquid of the test compound:
The test example compound was weighed, diluted to 10 mM with DMSO, and diluted to 100 µM with 100 mM phosphate buffer.

### 2.2 Experimental process:

1. Liver microsomes (40 µL), substrate (10 µL), and test compound (10 µL) were added to a 96-well plate and the mixture was pre-incubated for 3 min.
2. NADPH (40 µL) was added.
3. At 20 min, acetonitrile stop solution containing internal standard (300 µL) was added.
4. The mixture was centrifuged and the sample was loaded.

### 3 Experimental results:

| Compound | IC50 (µM) | | | |
|---|---|---|---|---|
| | 1A2 | 2C9 | 2C19 | 2D6 |
| Seltorexant | 46.3 | >100 | 9.1 | 61.6 |
| 6 | >100 | | | |
| 9 | >100 | >100 | 82.7 | >100 |
| 11 | >100 | >100 | 82.7 | >100 |

### Crystal form study

### 1.1 Experimental instruments

### 1.1.1 Some parameters of physical and chemical testing instruments

| | | |
|---|---|---|
| XRPD | Instrument model | BRUKER D8 ADVANCE |
| | Diffracted ray | CuK (40 kV, 25 mA) |
| | Scan rate | 0.02°/S (2θ value) |

| | Scan range | 4° - 40°(2θ value) |
|---|---|---|
| DSC | Instrument model | NETZSCH DSC 214 polyma |
| | Purge gas | Nitrogen gas |
| | Purge rate | 40 mL/min |
| | Heating rate | 10°C/min |
| | Temperature range | 25°C-350°C |
| | Tray type | Aluminium tray |
| TGA | Instrument model | NETZSCH TG 209 Tarsus |
| | Purge gas | Nitrogen gas |
| | Purge rate | 40 mL/min |
| | Heating rate | 10°C/min |
| | Temperature range | Room temperature-400°C |
| | Tray type | Al₂O₃ |

| | | |
|---|---|---|
| High performance liquid chromatograph | Thermo | U3000 |
| | Pump | LPG-3400SDN |
| | Sample injector | WPS-3000TSL |
| | Column oven | TCC-3000SD |
| | Detector | DAD-3000 |

### 1.1.2 Chromatographic conditions

| | | | |
|---|---|---|---|
| **Mobile phase** | A: 25 mM aqueous ammonium dihydrogen phosphate solution (pH2.0); B: acetonitrile (100%) | | |
| **Chromatographic column** | Aglient Bonus-RP (4.6*150 mm, 3.5 µm) | | |
| **Column temperature** | 35°C | | |
| **Sample tray temperature** | Room temperature | | |
| **Flow rate** | 1.0 mL/min | | |
| **Wavelength** | 254 nm | | |
| **Injection volume** | 5 µL | | |
| **Diluent** | Methanol/water (80 : 20, v/v) | | |
| **Gradient** | Time (min) | A% | B% |
| | 0.00 | 70.0 | 30.0 |
| | 8.00 | 30.0 | 70.0 |
| | 12.00 | 20.0 | 80.0 |
| | 12.01 | 70.0 | 30.0 |
| | 15.00 | 70.0 | 30.0 |

### 1. Preparation of different crystal forms

### 1.1 Preparation of the free base crystal form I of compound 9

About 20 mg of the free base was weighed and added to a 2 mL glass bottle, 100-200 µL of organic solvent (the organic solvent can be selected from MeOH, EtOH, EA, Acetone, IPA, IPAc, MEK, 2-Me-THF, MTBE, toluene, cyclohexane and heptane) was added, and the mixture was slurried at 50°C for 7 days. The obtained solid was centrifuged to remove the supernatant, and dried in vacuo at 40°C to afford the free base crystal form I. Detection and analysis showed that the free base crystal form I had an XRPD pattern as shown in FIG. 1, a DSC pattern as shown in FIG. 2, and a TGA pattern as shown in FIG. 3.

### 1.2 Preparation of the free base crystal form I of compound 9 (process and preparation method)

1) The crude product (15.3 g) was added to 20 mL of methanol to obtain a clear solution, water (30 mL) was added dropwise, and the mixture was stirred for 20 min to precipitate out a solid. Then, 6 mL of water (methanol : water = 1 : 1.8) was added dropwise, and the mixture was stirred for 16 h.
2) The mixture was filtered, and the filter cake was washed with a mixture of 4 mL of methanol +12 mL of water, and dried at 40°C to obtain a light brown solid.
3) The solid (7.7 g) was added to methanol (23 mL), and the mixture was heated to an outside temperature of 65°C until the solution was clear. The solution was cooled to 20°C and stirred for 0.5 h to precipitate out a solid, water (23 mL) was added dropwise, and the mixture was stirred for 4 h.
4) The mixture was filtered, and the filter cake was washed with a mixture of 10 mL of methanol +30 mL of water, and dried at 40°C to obtain a light brown solid.
5) The solid (7.3 g) was added to 22 mL (3V) of EA, and the mixture was heated to reflux and stirred for 1 h. 22 mL (3V) of n-heptane was added dropwise, and the mixture was stirred for 0.5 h, cooled to 20°C-24°C and stirred for 16 h.
6) The mixture was filtered, and the filter cake was washed with a mixture of 4 mL of EA+8 mL of n-heptane, dried at 40°C to afford the free base crystal form I as a light brown solid (6.5 g).

### 1.3 Preparation of the free base crystal form II of compound 9

About 20 mg of the free base was weighed and added to a 2 mL glass bottle, and 50-300 µL of solvent (the solvent can be selected from ACN, THF, DCM and water) was added. The mixture was slurried at 50°C for 7 days, and during this process, the obtained clear solution was evaporated at room temperature. The obtained solid was centrifuged to remove the supernatant, and dried in vacuo at 40°C to afford the free base crystal form II. Detection and analysis showed that the free base crystal form II had an XRPD pattern as shown in FIG. 4, a DSC pattern as shown in FIG. 5, and a TGA pattern as shown in FIG. 6.

### 2. Solid stability experiment

### 2.1 Experimental objective:

The compound physical and chemical stability at high temperature of 60°C, high humidity of 92.5% RH and both high temperature of 50°C and high humidity of 75% RH of the free base crystal form I and the free base crystal form II were investigated to provide a basis for the storage of the compounds.

### 2.2 Experimental scheme:

About 2 mg of the free base crystal form I and the free base crystal form II were taken respectively, and changes of related substances in the crystal forms at high temperature of 60°C, high humidity of 92.5% RH, and both high temperature of 50°C and high humidity of 75%RH for 7 days and 14 days were investigated. The changes of related substances were calculated by HPLC and the chromatographic peak area normalization method.

### Chromatography conditions:

| | | | |
|---|---|---|---|
| **Mobile phase** | A: 25 mM aqueous ammonium dihydrogen phosphate solution (pH2.07); B: acetonitrile (100%) | | |
| **Chromatographic column** | Aglient Bonus-RP (4.6*150 mm, 3.5 µm) | | |
| **Column temperature** | 35°C | | |
| **Sample tray temperature** | Room temperature | | |
| **Flow rate** | 1.0 mL/min | | |
| **Wavelength** | 254 nm | | |
| **Injection volume** | 5 µL | | |
| **Gradient** | Time (min) | A% | B% |
| | 0.00 | 70.0 | 30.0 |
| | 8.00 | 30.0 | 70.0 |
| | 12.00 | 20.0 | 80.0 |
| | 12.01 | 70.0 | 30.0 |
| | 15.00 | 70.0 | 30.0 |

### 2.3 Experimental results:

Physical and chemical stability results of the free base crystal form I:

| | | | |
|---|---|---|---|
| 60°C | 7 days | % Increase in total impurity | <0.1 |
| | | % Maximum increase in single impurity | <0.1 |
| | 14 days | % Increase in total impurity | <0.1 |
| | | % Maximum increase in single impurity | <0.1 |
| Room temperature, 92.5%RH | 7 days | % Increase in total impurity | <0.1 |
| | | % Maximum increase in single impurity | <0.1 |
| | 14 days | % Increase in total impurity | <0.1 |
| | | % Maximum increase in single impurity | <0.1 |
| 50°C, 75%RH | 7 days | % Increase in total impurity | <0.1 |
| | | % Maximum increase in single impurity | <0.1 |
| | 14 days | % Increase in total impurity | <0.1 |
| | | % Maximum increase in single impurity | <0.1 |

Physical and chemical stability results of the free base crystal form II:

| | | | |
|---|---|---|---|
| 60°C | 7 days | % Increase in total impurity | <0.10 |
| | | % Maximum increase in single impurity | <0.10 |
| | 14 days | % Increase in total impurity | <0.10 |
| | | % Maximum increase in single impurity | <0.10 |
| Room temperature, 92.5%RH | 7 days | % Increase in total impurity | <0.10 |
| | | % Maximum increase in single impurity | <0.10 |
| | 14 days | % Increase in total impurity | <0.11 |
| | | % Maximum increase in single impurity | <0.10 |
| 50°C, 75%RH | 7 days | % Increase in total impurity | <0.10 |
| | | % Maximum increase in single impurity | <0.10 |
| | 14 days | % Increase in total impurity | <0.10 |
| | | % Maximum increase in single impurity | <0.10 |

The above data showed that the free base crystal form I and the free base crystal form II of compound 9 were relatively stable after being placed for 7 days and 14 days under all conditions.

### 3 Hygroscopicity experiment

### 3.1 Experimental objective

The hygroscopicity of the crystal form I and the crystal form II of compound 9 were investigated under different relative humidity to provide a basis for the storage of the compounds.

### 3.2 Experimental scheme:

The crystal form I and the crystal form II of compound 9 were placed in saturated water vapor at different relative humidities to allow the compound to reach a dynamic equilibrium with the water vapor, and the percentage of weight gain after moisture absorption of the compound after equilibrium was calculated.

### 3.3 Experimental results:

The free base crystal form I had a weight gain after moisture absorption of about 0.3263% at 80%RH, indicating weak hygroscopicity. After 2 cycles of moisture absorption and desorption at 0%-95% relative humidity, there was no change in the XRPD pattern of the free base crystal form I, i.e., no crystal form transformation.

The free base crystal form II had a weight gain after moisture absorption of about 0.2053% at 80%RH, indicating weak hygroscopicity.

### 4. Solubility experiments in different media

### 4.1 Experimental objective

The solubility of the free base crystal form I and the free base crystal form II in media such as pH 1.0-8.0 USP buffer, artificial simulated gastric fluid (FaSSGF), fasted-state artificial simulated intestinal fluid (FaSSIF), fed-state artificial simulated intestinal fluid (FeSSIF) and pure water was compared to provide a basis for the evaluation of drugability.

### 4.2 Experimental scheme:

About 1 mg of the free base crystal form I and the free base crystal form II were respectively suspended in different media for 2 hours, and the thermodynamic solubility of the compound was determined at 37°C by HPLC and an external standard method.

### 4.3 Experimental results: The results were shown in Table 3.

**Table 3**

| Medium | Solubility of free base crystal form I (mg/mL) | Solubility of free base crystal form II |
|---|---|---|
| pH 1.0 | 0.799 | 0.599 |
| pH 2.0 | 0.141 | 0.089 |
| pH 3.0 | 0.087 | 0.055 |
| pH 4.0 | 0.073 | 0.051 |
| pH 5.0 | 0.088 | 0.050 |
| pH 6.0 | 0.070 | 0.050 |
| pH 7.0 | 0.075 | 0.047 |
| pH 8.0 | 0.058 | 0.047 |
| FaSSGF | 0.269 | 0.131 |
| FaSSIF | 0.247 | 0.060 |
| FeSSIF | 0.311 | 0.100 |
| FaSSCoF | 0.118 | 0.051 |
| FeSSCoF | 0.182 | 0.063 |
| H2O | 0.091 | 0.055 |
| pH 4.5 | 0.080 | 0.053 |
| pH 6.8 | 0.081 | 0.049 |

The above experimental results showed that the thermodynamic solubility of the free base crystal form I in each medium system was higher than that of the free base crystal form II, and the solubility of the free base crystal form I met the drug concentration required for local treatment.

### 5. PK studies in animals

### 5.1 Experimental objective:

SD rats were used as test animals to study the pharmacokinetic behaviour of the free base crystal form I and the free base crystal form II in rat (plasma) after single oral administration, to compare the changes in exposure. The pharmacokinetic of the free base crystal form after a single intravenous administration was studied, and the bioavailability of the free base crystal form I and the free base crystal form II after oral administration were calculated.

### 5.2 Experimental scheme:

The free base crystal form I was suspended with 0.5% CMCC-Na aqueous solution, and the free base crystal form I was suspended with 0.5% HPMC aqueous solution. After uniformity, the suspensions were administered intragastrically to rats in triplicate at a dosage of 5 mg/kg (30 mg/kg for suspension) for the free base crystal form I and a dosage of 30 mg/kg (suspension) for the free base crystal form II. The free base crystal form I was dissolved with 20% HP-β-CD aqueous solution to obtain a clear solution, and the clear solution was filtered through a filter membrane. The filtered solution was administered via injection to rats in triplicate.

### 5.3 Experimental results:

**Table 4 Animal PK experimental results of free base crystal form I and free base crystal form II**

| **Administration route: PO** | | | | **Administration route: IV** | |
|---|---|---|---|---|---|
| **Parameters** | **Crystal form II 30MPK** | **Crystal form I 30MPK** | **Crystal form I 5MPK** | **Parameters** | **Crystal form I 2MPK** |
| tₘₐₓ (h) | 2.0 | 2.0 | 0.5 | C0 (ng/mL) | 2914 |
| Cₘₐₓ (ng/mL) | 2517 | 6013 | 787 | AUC₀₋ₜ (ng/mL *h) | 2280 |
| AUC ₀₋ₜ (ng/mL *h) | 11810 | 28401 | 2389 | AUC _{0-∞} (ng/mL *h) | 2285 |
| AUC_{0-∞} (ng/mL *h) | 13588 | 30985 | 2414 | t_{1/2} (h) | 0.9 |
| t_{1/2} (h) | 2.4 | 1.9 | 1.2 | MRT _{0-∞} (h) | 0.9 |
| MRT _{0-∞} (h) | 4.3 | 3.8 | 2.2 | λz Cal. Time Range (h) | 0.25-8 |
| λz Cal. Time Range (h) | 4-8 | 4-8 | 1-8 | CL (mL/min/kg) | 15 |
| F% | 39.6 | 90.4 | 7.0 | Vss (L/kg) | 0.8 |
| Formulation | 0.5%HPMC | 0.5%CMC-Na | 0.5%CMC-Na | Formulation | 20%HP-B-CD |

It can be seen from the above data that the free base crystal form I shows a higher exposure than the free base crystal form II, and the exposure of the free base crystal form I at 30 mg/kg is significantly higher than that at 5 mg/kg.

## Claims

1. A crystal form of a compound represented by general formula (I), **characterized in that** the crystal form has a structure as shown below: 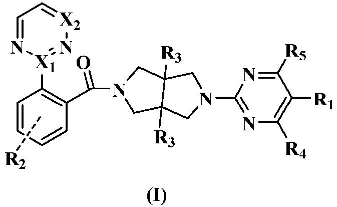 wherein
X₁ is CR₆ or N;
X₂ is CR₆ or absent;
R₁ is halogen;
R₂, R₃, R₄ or R₆ is independently selected from hydrogen, deuterium, halogen, amino, nitro, hydroxyl, mercapto, cyano, carboxyl, sulfo, oxo, thio, alkyl, deuteroalkyl, haloalkyl, hydroxyalkyl, alkoxy, haloalkoxy, hydroxyalkoxy, alkenyl, alkynyl, cycloalkyl, heterocyclyl, aryl or heteroaryl, respectively, wherein the amino, alkyl, deuteroalkyl, haloalkyl, hydroxyalkyl, alkoxy, haloalkoxy, hydroxyalkoxy, alkenyl, alkynyl, cycloalkyl, heterocyclyl, aryl and heteroaryl can be optionally further substituted;
R₂, R₃, R₄ or R₆ is each independently preferably hydrogen, deuterium, halogen, amino, nitro, hydroxyl, mercapto, cyano, carboxyl, sulfo, oxo, thio, C₁₋₈ alkyl, C₁₋₈ deuteroalkyl, C₁₋₈ haloalkyl, C₁₋₈ hydroxyalkyl, C₁₋₈ alkoxy, C₁₋₈ haloalkoxy, C₁₋₈ hydroxyalkoxy, C₂₋₈ alkenyl, C₂₋₈ alkynyl, C₃₋₁₂ cycloalkyl, 3- to 12-membered heterocyclyl, C₆₋₁₄ aryl or 5- to 14-membered heteroaryl, wherein the amino, C₁₋₈ alkyl, C₁₋₈ deuteroalkyl, C₁₋₈ haloalkyl, C₁₋₈ hydroxyalkyl, C₁₋₈ alkoxy, C₁₋₈ haloalkoxy, C₁₋₈ hydroxyalkoxy, C₂₋₈ alkenyl, C₂₋₈ alkynyl, C₃₋₁₂ cycloalkyl, 3- to 12-membered heterocyclyl, C₆₋₁₄ aryl and 5- to 14-membered heteroaryl are optionally further substituted with one or more substituents of deuterium, halogen, amino, nitro, hydroxyl, mercapto, cyano, carboxyl, sulfo, oxo, thio, C₁₋₈ alkyl, C₁₋₈ deuteroalkyl, C₁₋₈ haloalkyl, C₁₋₈ hydroxyalkyl, C₁₋₈ alkoxy, C₁₋₈ haloalkoxy, C₂₋₈ alkenyl, C₂₋₈ alkynyl, C₃₋₁₂ cycloalkyl, 3- to 12-membered heterocyclyl, C₆₋₁₄ aryl and 5- to 14-membered heteroaryl,
more preferably hydrogen, deuterium, halogen, amino, nitro, hydroxyl, mercapto, cyano, carboxyl, sulfo, oxo, thio, C₁₋₆ alkyl, C₁₋₆ deuteroalkyl, C₁₋₆ haloalkyl, C₁₋₆ hydroxyalkyl, C₁₋₆ alkoxy, C₁₋₆ haloalkoxy, C₁₋₆ hydroxyalkoxy, C₂₋₆ alkenyl, C₂₋₆ alkynyl, C₃₋₈ cycloalkyl, 3- to 8-membered heterocyclyl, C₆₋₁₀ aryl or 5- to 10-membered heteroaryl, wherein the amino, C₁₋₆ alkyl, C₁₋₆ deuteroalkyl, C₁₋₆ haloalkyl, C₁₋₆ hydroxyalkyl, C₁₋₆ alkoxy, C₁₋₆ haloalkoxy, C₁₋₆ hydroxyalkoxy, C₂₋₆ alkenyl, C₂₋₆ alkynyl, C₃₋₈ cycloalkyl, 3- to 8-membered heterocyclyl, C₆₋₁₀ aryl and 5- to 10-membered heteroaryl are optionally further substituted with one or more substituents of deuterium, halogen, amino, nitro, hydroxyl, mercapto, cyano, carboxyl, sulfo, oxo, thio, C₁₋₆ alkyl, C₁₋₆ deuteroalkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₈ cycloalkyl, 3- to 8-membered heterocyclyl, C₆₋₁₀ aryl and 5- to 10-membered heteroaryl;
alternatively, any two or more of R₂, R₃ and R₄ and the atom to which they are attached are connected to form cycloalkyl, heterocyclyl, aryl or heteroaryl, wherein the cycloalkyl, heterocyclyl, aryl and heteroaryl can be optionally further substituted;
R₅ is selected from hydroxyalkyl or haloalkyl, which can be optionally further substituted, wherein the hydroxyalkyl is preferably 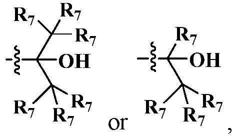 and the haloalkyl is preferably 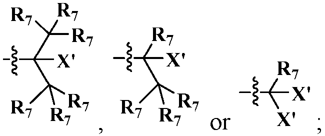
X' is halogen;
R₇ is independently selected from hydrogen, deuterium, halogen, amino, nitro, hydroxyl, mercapto, cyano, carboxyl, sulfo, oxo, thio, alkyl, deuteroalkyl, haloalkyl, hydroxyalkyl, alkoxy, haloalkoxy, hydroxyalkoxy, alkenyl, alkynyl, cycloalkyl, heterocyclyl, aryl or heteroaryl, respectively, wherein the amino, alkyl, deuteroalkyl, haloalkyl, hydroxyalkyl, alkoxy, haloalkoxy, hydroxyalkoxy, alkenyl, alkynyl, cycloalkyl, heterocyclyl, aryl and heteroaryl can be optionally further substituted.

2. The crystal form according to claim 1, **characterized in that** the general formula (I) is further as represented by general formula (II): 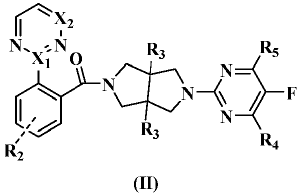 wherein
X₁ is CH or N;
X₂ is CH or absent;
R₂, R₃ or R₄ is independently selected from hydrogen, deuterium, halogen, amino, hydroxyl, C₁₋₃ alkyl, C₁₋₃ deuteroalkyl, C₁₋₃ haloalkyl, C₁₋₃ hydroxyalkyl, C₁₋₃ alkoxy, C₁₋₃ haloalkoxy or C₁₋₃ hydroxyalkoxy, respectively;
R₅ is selected from

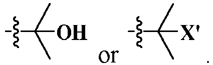

3. The crystal form according to claim 1 or 2, **characterized in that**
when X₂ is absent, substituent R₂ is preferably halogen, wherein the halogen is preferably located at an ortho or meta position of triazole, more preferably at an ortho position of triazole, and
the halogen is preferably fluorine;
when X₂ is CR₆, substituent R₂ is preferably halogen, wherein the halogen is preferably located at an ortho or meta position of pyrimidine, more preferably at a meta position of pyrimidine, and/or at an ortho position of carbonyl, and
the halogen is preferably fluorine.

4. The crystal form according to claim 1, **characterized in that** the general formula (I) is selected from the following compounds:

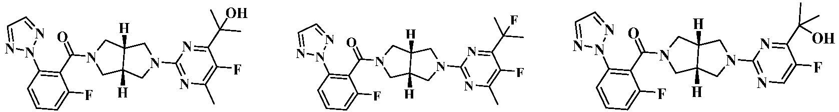

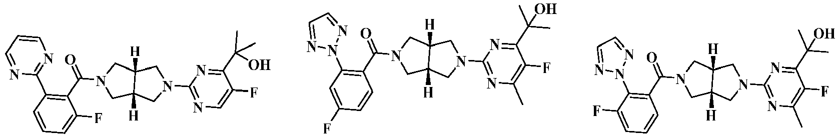

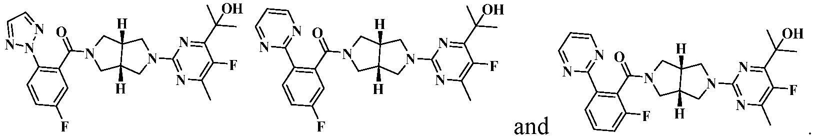

5. A crystal form of ((3aR,6aS)-5-(5-fluoro-4-(2-hydroxyprop-2-yl)pyrimidin-2-yl)hexahydropyrrolo[3,4-c]pyrrol-2(1H)-yl)(2-fluoro-6-(pyrimidin-2-yl)phenyl)methanone, **characterized in that** the crystal form comprises crystal form I and crystal form II, wherein
the X-ray powder diffraction pattern of the crystal form I has a diffraction peak at 2θ of 8.6±0.2°, or has a diffraction peak at 2θ of 9.2±0.2°, or has a diffraction peak at 2θ of 10.2±0.2°, or has a diffraction peak at 2θ of 10.8±0.2°, or has a diffraction peak at 2θ of 12.0±0.2°, or has a diffraction peak at 2θ of 12.8±0.2°, or has a diffraction peak at 2θ of 13.7±0.2°, or has a diffraction peak at 2θ of 14.2±0.2°, or has a diffraction peak at 2θ of 15.5±0.2°, or has a diffraction peak at 2θ of 16.6±0.2°, or has a diffraction peak at 2θ of 17.3±0.2°, or has a diffraction peak at 2θ of 18.4±0.2°, or has a diffraction peak at 2θ of 19.0±0.2°, or has a diffraction peak at 2θ of 19.5±0.2°, or has a diffraction peak at 2θ of 20.5±0.2°, or has a diffraction peak at 2θ of 21.3±0.2°, or has a diffraction peak at 2θ of 26.5±0.2°; or has a diffraction peak at 2θ of 28.6±0.2°; preferably, the X-ray powder diffraction pattern optionally has diffraction peaks at 2, 4, 6, 8 or 10 of the above-mentioned 2θ; and
the X-ray powder diffraction pattern of the crystal form II has a diffraction peak at 2θ of 9.9±0.2°, or has a diffraction peak at 2θ of 10.8±0.2°, or has a diffraction peak at 2θ of 13.2±0.2°, or has a diffraction peak at 2θ of 14.9±0.2°, or has a diffraction peak at 2θ of 16.4±0.2°, or has a diffraction peak at 2θ of 17.1±0.2°, or has a diffraction peak at 2θ of 17.9±0.2°, or has a diffraction peak at 2θ of 19.3±0.2°, or has a diffraction peak at 2θ of 19.8±0.2°, or has a diffraction peak at 2θ of 20.2±0.2°, or has a diffraction peak at 2θ of 20.5±0.2°, or has a diffraction peak at 2θ of 21.0±0.2°, or has a diffraction peak at 2θ of 21.9±0.2°, or has a diffraction peak at 2θ of 25.8±0.2°, or has a diffraction peak at 2θ of 26.5±0.2°, or has a diffraction peak at 2θ of 27.5±0.2°; preferably, the X-ray powder diffraction pattern optionally has diffraction peaks at 2, 4, 6, 8 or 10 of the above-mentioned 2θ.

6. The crystal form according to claim 5, **characterized in that** the X-ray powder diffraction pattern of the crystal form I has at least a diffraction peak at one or more of 2θ of 9.2±0.2°, 10.2±0.2°, 16.6±0.2° or 18.4±0.2°, preferably at 2 to 4 of the above-mentioned 2θ, more preferably at 3 or 4 of the above-mentioned 2θ, and most preferably at 4 of the above-mentioned 2θ; optionally, further, the X-ray powder diffraction pattern can also have a diffraction peak at one or more of 2θ of 12.8±0.2°, 17.3±0.2°, 19.0±0.2°, 21.3±0.2° or 26.5±0.2°, preferably at 2, 3, 4 or 5 of the above-mentioned 2θ;
the X-ray powder diffraction pattern of the crystal form II has at least a diffraction peak at one or more of 2θ of 16.4±0.2°, 17.1±0.2°, 17.9±0.2° or 25.8±0.2°, preferably at 2 to 4 of the above-mentioned 2θ, more preferably at 3 or 4 of the above-mentioned 2θ, and most preferably at 4 of the above-mentioned 2θ; optionally, further, the X-ray powder diffraction pattern can also have a diffraction peak at one or more of 2θ of 10.8±0.2°, 14.9±0.2°, 19.3±0.2°, 21.9±0.2° or 26.5±0.2°, preferably at 2, 3, 4 or 5 of the above-mentioned 2θ.

7. The crystal form according to claim 5, **characterized in that** the X-ray powder diffraction pattern of the crystal form I has characteristic peaks at 2θ of 9.2±0.2° and 18.4±0.2°; preferably, the X-ray powder diffraction pattern also has characteristic peaks at 2θ of 10.2±0.2°, 16.6±0.2°, 17.3±0.2° and 19.0±0.2°; more preferably, the X-ray powder diffraction pattern also has characteristic peaks at 2θ of 12.8±0.2° and 21.3±0.2°; further preferably, the X-ray powder diffraction pattern also has characteristic peaks at 2θ of 26.5±0.2° and 28.6±0.2°, more further preferably, the X-ray powder diffraction pattern also has characteristic peaks at 8.6±0.2°, 10.8±0.2°, 12.0±0.2°, 13.7±0.2°, 14.2±0.2°, 15.5±0.2°, 19.5±0.2°, 20.5±0.2°, 23.1±0.2°, 27.8±0.2° and 32.1±0.2°; most preferably, the X-ray powder diffraction pattern thereof is basically as shown in FIG. 1; the DSC pattern thereof is basically as shown in FIG. 2, and the TGA pattern thereof is basically as shown in FIG. 3;
the X-ray powder diffraction pattern of the crystal form II has characteristic peaks at 2θ of 16.4±0.2° and 25.8±0.2°; preferably, the X-ray powder diffraction pattern also has characteristic peaks at 2θ of 17.1±0.2°, 17.9±0.2°, 21.9±0.2° and 26.5±0.2°; more preferably, the X-ray powder diffraction pattern also has characteristic peaks at 2θ of 10.8±0.2° and 14.9±0.2°; further preferably, the X-ray powder diffraction pattern also has characteristic peaks at 2θ of 9.9±0.2° and 19.3±0.2°, more further preferably, the X-ray powder diffraction pattern also has characteristic peaks at 13.2±0.2°, 19.8±0.2°, 20.2±0.2°, 20.5±0.2°, 21.0±0.2° and 27.5±0.2°; most preferably, the X-ray powder diffraction pattern thereof is basically as shown in FIG. 4; the DSC pattern thereof is basically as shown in FIG. 5, and the TGA pattern thereof is basically as shown in FIG. 6.

8. A method for preparing the crystal form of the compound according to any one of claims 1-7, **characterized in that** the method specifically comprises the following steps:
1) weighing an appropriate amount of a free base, and suspending the free base with a poor solvent preferably at a suspension density of 50-200 mg/mL;
2) shaking a suspension obtained above preferably at a temperature of 0°C-50°C for preferably 1-10 days;
3) quickly centrifuging the suspension, removing a supernatant, and drying the remaining solid to a constant weight to afford a target product;
wherein
the poor solvent is selected from acetone, ethyl acetate, isopropyl acetate, acetonitrile, ethanol, tetrahydrofuran, 2-methyltetrahydrofuran, dichloromethane, 1,4-dioxane, benzene, toluene, isopropanol, n-butanol, isobutanol, N,N-dimethylformamide, N,N-dimethylacetamide, n-propanol, tert-butanol, 2-butanone or 3-pentanone, methyl tert-butyl ether, water, heptane or n-pentane; preferably isopropyl acetate, toluene, methyl tert-butyl ether, water, heptane or n-pentane;
or
1) weighing an appropriate amount of a free base, and dissolving the free base with a good solvent;
2) adding an anti-solvent to a solution obtained above and performing stirring until a solid precipitates, preferably at a temperature of 0°C-25°C;
3) quickly centrifuging a suspension obtained above, removing a supernatant, and drying the remaining solid to a constant weight to afford a target product;
wherein
the good solvent is selected from methanol, acetone, ethyl acetate, acetonitrile, ethanol, tetrahydrofuran, dichloromethane, 1,4-dioxane, benzene, toluene, isopropanol, n-butanol, isobutanol, N,N-dimethylformamide, N,N-dimethylacetamide, n-propanol, tert-butanol, 2-butanone or 3-pentanone; preferably methanol, dichloromethane or ethyl acetate;
the anti-solvent is selected from heptane, water, methyl tert-butyl ether, cyclohexane or isopropyl acetate;
or
1) weighing an appropriate amount of a free base, and heating and dissolving the free base with a good solvent;
2) quickly placing a solution obtained above under low temperature and stirring the solution until a solid precipitates, wherein the temperature is preferably -10°C-5°C;
3) quickly centrifuging a suspension obtained above, removing a supernatant, and drying the remaining solid to a constant weight to afford a target product;
wherein
the good solvent is selected from methanol, acetone, ethyl acetate, acetonitrile, ethanol, tetrahydrofuran, dichloromethane, 1,4-dioxane, benzene, toluene, isopropanol, n-butanol, isobutanol, N,N-dimethylformamide, N,N-dimethylacetamide, n-propanol, tert-butanol or 2-butanone or tetrahydrofuran; preferably acetone.

9. A pharmaceutical composition comprising a therapeutically-effective amount of the crystal form of the compound according to any one of claims 1-7, and one or more pharmaceutically acceptable carriers, diluents or excipients.

10. Use of the crystal form according to any one of claims 1-7 or the pharmaceutical composition according to claim 9 as an orexin receptor antagonist in the preparation of a drug for treating nervous system disease, **characterized in that** the use in the preparation of an OX2R selective receptor antagonist is preferred, and
more preferably, the nervous system disease is selected from insomnia, depression, anxiety or drug addiction, more preferably major depressive disorder, primary and secondary insomnia, or depression accompanied by insomnia.
